# FASCICULE DE BREVET EUROPEEN

(11) **EP 2 140 858 B1**
(45) Date de publication et mention de la délivrance du brevet: **05.10.2011**
(21) Numéro de dépôt: 09162531.9
(22) Date de dépôt: 12.06.2009
(51) Int. Cl.: A61K 8/49, A61K 8/92, A61Q 1/04, A61Q 3/02, A61Q 1/06, A61Q 1/02

(54) **Composition cosmétique comprenant un composé capable d'établir des liaisons hydrogène, et procédé de traitement cosmétique**
Kosmetische Zusammensetzung, die eine Komponente enthält, die in der Lage ist, Wasserstoffverbindungen zu bilden, und kosmetisches Behandlungsverfahren
Cosmetic composition comprising a compound capable of creating hydrogen bonds, and cosmetic treatment method

(30) Priorité: 04.07.2008 FR 0854556
(43) Date de publication de la demande: 06.01.2010
(73) Titulaire: L'Oréal, 75008 Paris (FR)
(72) Inventeur: Chodorowski-Kimmes, Sandrine, 60300 Senlis (FR); Rodriguez, Ivan, 60290 Cauffry (FR)
(74) Mandataire: Dodin, Catherine

(56) Documents cités:
- EP-A- 1 097 699
- EP-A- 1 797 867
- WO-A-2006/118460
- US-A- 5 707 612

## Description

La présente invention concerne une composition cosmétique, notamment de soin, de traitement et/ou de maquillage des matières kératiniques, comprenant de nouveaux composés susceptibles d'établir des liaisons hydrogène avec des groupes de jonction partenaires, lesdits composés permettant d'obtenir un effet 'longue durée' d'un dépôt formé sur lesdites matières kératiniques, ledit effet 'longue durée' pouvant être lié à la tenue de la couleur, à la tenue de la brillance, à la tenue en tant que telle, associée ou non à un effet 'non collant' et/ou 'non transfert'.

Il existe de nombreuses compositions cosmétiques pour lesquelles des propriétés de brillance du film déposé, après application sur les matières kératiniques, sont souhaitées. On peut citer par exemple les rouges à lèvres ou les vernis à ongles. Afin d'obtenir un tel résultat, il est possible d'associer des matières premières particulières, notamment des lanolines, avec des huiles dites brillantes telles que les polybutènes, ou des esters d'acide ou d'alcool gras dont le nombre de carbone est élevé; ou bien certaines huiles végétales; ou encore des esters résultants de l'estérification partielle ou totale d'un composé aliphatique hydroxylé avec un acide aromatique, comme décrit dans la demande de brevet EP1097699.

Pour améliorer la brillance et la tenue dans le temps du film déposé, il a également été proposé d'utiliser des huiles de type triglycérides, l'huile de ricin en l'occurrence, fonctionnalisées par de l'isophorone diisocyanate (IPDI), ainsi que cela est décrit dans US5707612. La fonctionnalisation par l'IPDI améliore sensiblement la tenue et la brillance de l'huile de ricin; les huiles ainsi réticulées trouvent une application notamment dans le domaine des rouges à lèvres.

Toutefois, on a constaté que ces huiles réticulées, si elles apportaient de la tenue et de la brillance au dépôt, ne permettaient pas d'obtenir sur le substrat kératinique, un dépôt homogène et cohésif, qui forme un film uniforme, et qui soit en outre non collant et sans transfert.

La présente invention a pour but de proposer des compositions cosmétiques qui permettent d'obtenir un tel dépôt filmogène uniforme sur le substrat, ledit film alliant la brillance, la tenue de la brillance et la tenue de la composition, tout en étant non collant et particulièrement confortable à porter.

La présente invention a donc pour objet une composition cosmétique comprenant, dans un milieu cosmétiquement acceptable, un composé susceptible d'être obtenu par réaction entre :
- une huile portant au moins une fonction réactive nucléophile et/ou électrophile, et
- un groupe de jonction capable d'établir des liaisons hydrogène avec un ou plusieurs groupes de jonction partenaires, chaque appariement d'un groupe de jonction faisant intervenir au moins 3 liaisons hydrogène, ledit groupe de jonction portant au moins une fonction réactive susceptible de réagir avec la fonction réactive portée par l'huile, ledit groupe de jonction comprenant au moins en outre un motif de formule (I) ou (II) tel que défini ci-après.

Les huiles fonctionnalisées selon la présente invention se présentent sous forme d'un solide; ceci permet notamment de former un matériau non collant, qui ne transfère pas au doigt une fois appliqué sur un substrat kératinique; ceci n'est pas le cas avec les huiles fonctionnalisées de l'art antérieur, notamment telles que décrites dans US5707612, qui se présentent sous forme d'un liquide, plus ou moins visqueux, et qui forment un matériau collant, qui transfère au doigt après application sur un substrat.

Par ailleurs, on a constaté que la réticulation à travers quatre liaisons hydrogène, par l'intermédiaire des groupes uréïdopyrimidone, pouvait permettre d'augmenter la force de cette réticulation, et donc améliorer la tenue de l'effet cosmétique recherché, tout particulièrement la tenue du dépôt ou de la brillance.

De plus, les composés, ou huiles fonctionnalisées, selon l'invention sont aisément véhiculables dans les milieux cosmétiques usuels, notamment les milieux huileux cosmétiques usuels.

Ils sont avantageusement compatibles avec les huiles présentes habituellement dans les compositions cosmétiques, et possèdent également de bonnes propriétés de dispersion des pigments ou des charges.

Ils sont aisément véhiculables dans les milieux solvants ou huileux cosmétiques, notamment les huiles, les alcools gras et/ou les esters gras, ce qui facilite leur mise en oeuvre dans le domaine cosmétique, notamment dans les rouges à lèvres. Ils présentent une solubilité convenable dans des milieux huileux cosmétiques variés, tels que les huiles végétales, les alcanes, les esters qu'ils soient courts de type acétate de butyle ou d'éthyle, ou gras, les alcools gras et tout particulièrement dans les milieux comprenant de l'isododécane, du Parléam, de l'isononanoate d'isononyle, de l'octyldodécanol, et/ou un benzoate d'alkyle en C12-C15.

Les compositions cosmétiques selon l'invention présentent par ailleurs une bonne applicabilité et une bonne couvrance; une bonne adhérence sur le support, que cela soit sur l'ongle, les cils, la peau ou les lèvres; une flexibilité et une résistance du film adéquates, ainsi qu'un excellent niveau de brillance durable. Les propriétés de confort et de glissant sont également très satisfaisantes.

Les composés selon l'invention sont susceptibles d'être obtenus par réaction entre :
- d'une part au moins une huile portant au moins une fonction réactive, nucléophile et/ou électrophile, et
- d'autre part au moins un groupe de jonction capable d'établir des liaisons hydrogène avec un ou plusieurs groupes de jonction partenaires, chaque appariement d'un groupe de jonction faisant intervenir au moins 3 liaisons hydrogène, ledit groupe de jonction portant au moins une fonction réactive susceptible de réagir avec la fonction réactive portée par l'huile, ledit groupe de jonction comprenant au moins un motif de formule (I) ou (II) telle que définie ci-après.

De préférence, les composés selon l'invention sont susceptibles d'être obtenus par réaction entre :
- d'une part au moins une huile portant au moins une fonction réactive nucléophile choisie parmi OH et NH₂, et
- d'autre part au moins un groupe de jonction capable d'établir des liaisons hydrogène avec un ou plusieurs groupes de jonction partenaires, chaque appariement d'un groupe de jonction faisant intervenir au moins 3 liaisons hydrogène, ledit groupe de jonction portant au moins une fonction réactive isocyanate ou imidazole, ledit groupe de jonction comprenant au moins un motif de formule (I) ou (II) telle que définie ci-après.

Au final, les composés selon l'invention comportent donc au moins une partie (HB) provenant de l'huile et au moins une partie (G) provenant du groupe de jonction, ladite partie (G) comprenant au moins un motif de formule (I) ou (II).
Notamment, lesdites parties (HB) et (G) sont reliées par une liaison covalente, notamment peuvent être reliées par une liaison covalente formée lors de la réaction entre les fonctions réactives OH et/ou NH₂ portées par l'huile et les fonctions isocyanate portées par le groupe de jonction; ou bien entre les fonctions réactives NH₂ portées par l'huile et les fonctions isocyanate ou imidazole portées par le groupe de jonction.

On peut donc notamment schématiser l'obtention préférentielle des composés selon l'invention par la réaction chimique entre les entités suivantes :
(HB)-(OH)ₘ(NH₂)ₙ + (G)-(NCO)ₚ ou bien
(HB)-(OH)ₘ(NH₂)ₙ + (G)-(imidazole)ₚ avec m, n et p étant des entiers non nuls.

L'huile susceptible d'être employée pour préparer le composé selon l'invention, qui de préférence peut être schématisée (HB)-(OH)ₘ(NH₂)ₙ, est un corps gras ou un mélange de corps gras, non cristallin à 25°C, liquide à température ambiante et sous pression atmosphérique (25°C, 1 atm.); de préférence apolaire, voire de préférence non soluble à l'eau.

Par liquide, on entend que la viscosité du composé est inférieure ou égale à 2500 centipoises, à 110°C, 1 atm., mesurée avec un rhéomètre Brookfield DV-I ou Brookfield Cap 1000+, l'homme de l'art choisissant l'appareil adapté à la mesure de viscosité.
Par apolaire, on entend un compose dont la valeur de HLB (hydrophile lipophile balance) est faible; notamment inférieure ou égale à 8, de préférence inférieure ou égale à 4, et encore mieux inférieure ou égale à 2; préférentiellement, la valeur de HLB doit être suffisamment peu élevée pour permettre d'obtenir un matériau supramoléculaire qui n'est pas, ou pas trop, hygroscopique.
Par non soluble, on entend que la fraction d'huile qui peut se dissoudre dans l'eau, à 25°C, 1 atm., est inférieure à 5% en poids (soit 5 g d'huile dans 100 ml d'eau); de préférence inférieure à 3%.
Par corps gras, on entend notamment mais pas exclusivement, un composé hydrocarboné comportant une ou plusieurs chaînes alkyle, linéaires, cycliques, ramifiées, saturées ou non, ayant au moins 6 atomes de carbone et pouvant comporter des groupes polaires comme un groupe acide, un hydroxyle ou polyol, amine, amide, acide phosphorique, phosphate, ester, éther, urée, carbamate, thiol, thioéther, thioester, cette chaîne pouvant comporter jusqu'à 100 atomes de carbone.

De préférence, l'huile susceptible d'être employée pour préparer le composé selon l'invention est une huile brillante, c'est-à-dire ayant un indice de réfraction supérieur ou égal à 1,46 à 25°C, en particulier compris entre 1,46 à 1,55 (l'indice de réfraction étant défini par rapport à la raie D du sodium, à 25°C).

De préférence, l'huile susceptible d'être employée pour préparer le composé selon l'invention est une huile non volatile. Par "huile non volatile", on entend une huile susceptible de rester sur les matières kératiniques à température ambiante et pression atmosphérique au moins plusieurs heures, et notamment ayant une pression de vapeur inférieure à 10⁻³ mm de Hg (0,13 Pa).
De préférence, l'huile a une masse molaire (Mw) comprise entre 150 et 6000, notamment entre 170 et 4000, voire entre 180 et 2000, préférentiellement entre 200 et 1500, et encore mieux entre 220 et 800 g/mol.

L'huile susceptible d'être utilisée dans le cadre de la présente invention porte au moins une fonction réactive susceptible de réagir avec la fonction réactive portée sur le groupe de jonction, notamment susceptible de réagir chimiquement avec les groupes isocyanates ou imidazole portés par le groupe de jonction; de préférence, cette fonction est une fonction OH ou NH₂. De préférence, l'huile ne comporte que des fonctions OH, en particulier 1 à 3 fonctions OH, préférentiellement des fonctions OH primaire ou secondaires, et encore mieux uniquement primaires.
L'huile selon la présente invention est de préférence une huile carbonée, notamment hydrocarbonée, qui, outre la fonction réactive susceptible de réagir avec le groupe de jonction, peut comporter des atomes d'oxygène, d'azote, de soufre et/ou de phosphore. L'huile est très préférentiellement choisie parmi les huiles cosmétiquement acceptables.

L'huile susceptible d'être employée dans le cadre de la présente invention peut être choisie parmi :
(i) les alcools gras, comprenant 6 à 50 atomes de carbone, linéaires, ramifiés ou cycliques, saturés ou insaturés, comprenant 1 ou plusieurs OH; éventuellement comprenant un ou plusieurs NH₂.
   On peut en particulier citer :
   - les monoalcools linéaires ou ramifiés en C6-C50, notamment en C6-C32, en particulier en C8-C28, saturés ou insaturés, et notamment l'alcool isostéarylique, l'alcool cétylique, l'alcool oléique, l'alcool oléylique, l'alcool isopalmitoylique, le butyl-2 octanol, l'hexyl-2 décanol, l'octyl-2 décanol, l'octyl-2 dodécanol, l'octyl-2 tétradécanol, le décyl-2 tétradécanol, le dodécyl-2 hexadécanol, et notamment les alcools vendus sous la dénomination Jarcol par la société Jarchem Industries, tels que le Jarcol I-12, le Jarcol I-16, le Jarcol I-20 et le Jarcol I-24;
   - les diols linéaires ou ramifiés en C6-C50, notamment en C6-C40, en particulier en C8-C38, saturés ou insaturés, et notamment ramifié en C32-36, et en particulier le produit commercial Pripol 2033 d'Uniqema;
   - les triols linéaires ou ramifiés en C6-C50, notamment en C6-C32, en particulier en C8-C28, saturés ou insaturés, et notamment le phytantriol;
(ii) les esters et les éthers portant au moins un groupe OH libre, et notamment les esters et éthers partiels de polyol, et les esters d'acide carboxylique hydroxylé.
   Par ester partiel de polyol, on entend les esters préparés par estérification d'un polyol avec un acide carboxylique, substitué ou non, la réaction n'étant pas totale, c'est-à-dire pas effectuée sur la totalité des OH libres du polyol; au final, l'ester comporte donc encore au moins un OH libre.
   De préférence, l'acide carboxylique est un monoacide. On peut également employer un mélange d'acides carboxyliques, notamment monocarboxyliques.
   Par éther partiel de polyol, on entend les éthers préparés par éthérification d'un polyol, sur lui-même ou avec au moins un autre alcool, mono ou polyhydroxylé, de préférence monoalcool, la réaction d'éthérification n'étant pas totale, c'est-à-dire pas effectuée sur la totalité des OH libres du polyol; au final, l'éther comporte encore au moins un OH libre.
   Par ester d'acide carboxylique hydroxylé, on entend les esters (mono et poly) préparés par réaction entre un acide carboxylique portant au moins une fonction OH, et un ou plusieurs alcools (mono ou poly), de préférence monoalcool, la réaction pouvant être totale ou partielle (effectuée sur tout ou partie des OH libres de l'alcool).
   Parmi les polyols susceptibles d'être utilisés pour préparer les esters ou éthers ci-dessus, on peut citer le propylène glycol, le glycérol, le néopentylglycol, le triméthylolpropane, le triméthyloléthane, les polyglycérols et notamment polyglycérol-2, polyglycérol-3 et polyglycérol-10; l'érythritol, le dipentaérythritol, le pentaérythritol, le di-triméthylolpropane, le phytantriol, le saccharose, le glucose, le méthylglucose, le sorbitol, le fructose, le xylose, le mannitol, la glucosamine; ainsi que les dimères diols notamment obtenus à partir d'acides gras dimères, notamment les diols ramifiés en C32-C38, notamment C36, aliphatiques et/ou alycycliques, tels que ceux définis dans l'article Hofer et al. European Coating Journal (mars 2000), pages 26-37; et leurs mélanges.
   Parmi les monoalcools susceptibles d'être utilisés pour préparer les esters ou éthers ci-dessus, on peut citer les alcools en C3-C50, linéaires ou ramifiés, de préférence ramifiés, et notamment le 2-éthylhexanol, l'octanol, l'alcool isostéarylique, et leurs mélanges.
   Parmi les acides carboxyliques susceptibles d'être utilisés pour préparer les esters ou éthers ci-dessus, on peut citer les monoacides ayant 6 à 50 atomes de carbone et les diacides ayant 3 à 12 atomes de carbone, linéaires ou ramifiés, saturés ou insaturés, parmi lesquels on peut citer l'acide octyldodécylique, l'acide hexyldécylique, l'acide éthylhexylique, l'acide isostéarique, l'acide nonanoïque, l'acide isononanoïque, l'acide arachidique, l'acide stéarique, l'acide palmitique, l'acide oléique, l'acide oxalique, l'acide adipique, l'acide succinique, l'acide fumarique, l'acide maléique, l'acide caprique, l'acide hexanedioïque, l'acide décylique, l'acide décanoïque, et leurs mélanges.
   Parmi les acides carboxyliques hydroxylés susceptibles d'être utilisés pour préparer les esters ou éthers ci-dessus, on peut citer les acides monohydroxylés ou polyhydroxylés, de préférence monohydroxylés; ayant 4 à 28 atomes de carbone par exemple, et notamment l'acide 12-hydroxystéarique, l'acide ricinoléïque, l'acide malique, l'acide lactique, l'acide citrique; et leurs mélanges.
   Ainsi, l'huile susceptible d'être employée dans la présente invention peut être choisie parmi, seuls ou en mélange:
   - les esters partiels de pentaérythritol, et notamment l'adipate de pentaérythrityle, le caprate de pentaérythrityle, le succinate de pentaérythrityle, le tétraisononanoate de pentaérythrityle, le triisononanoate de pentaérythrityle, le tétraisostéarate de pentaérythrityle, le triisostéarate de pentaérythrityle, le tétradécyl-2 tétradécanoate de pentaérythrityle, le tétraéthyl hexanoate de pentaérythrityle, le tétraoctyl dodécanoate de pentaérythrityle.
   - les diesters, triesters, tetraesters ou pentaesters de dipentaérythritol, et notamment le dipentaérythrityle pentaisononanoate, le dipentaérythrityle pentaisostéarate, le dipentaérythrityle tétraisostéarate, le dipentaérythrityle tri(polyhydroxystéarate);
   - les mono et di-esters de triméthylolpropane comme le triméthylolpropane mono-isostéarate, le triméthylolpropane di-isostearate, le triméthylolpropane monoethyl-2 hexylate, le triméthylolpropane diethyl-2 hexylate;
   - les mono-, di- et tri-esters de di-triméthylolpropane comme le di-triméthylolpropane di-isostearate, le di-triméthylolpropane tri-isostearate, le di-triméthylolpropane tri-ethyl hexanoate;
   - les mono-esters ou poly-esters partiels de glycérol ou de polyglycérols, et notamment :
      - le di-isostearate de glycérol, le di-isononanoate de glycérol,
      - les mono-, di- et tri-esters de polyglycérol-2; par exemple avec l'acide isostéarique, l'acide ethyl-2 hexylique et/ou l'acide isononanoïque; et notamment le polyglycéryl-2-isostéarate; le polyglycéryl-2-diisostéarate; le triisostéarate de polyglycéryl-2; le polyglycéryl-2-nonaisostearate; le polyglycéryl-2-nonanoate;
      - les mono-, di-, tri- ou tétra-esters de polyglycérol-3; par exemple avec soit l'acide isostéarique, l'acide ethyl-2 hexylique et/ou l'acide isononanoïque; et notamment le polyglycéryl-3-isostéarate, le polyglycéryl-3-diisostéarate; le triisostéarate de polyglycéryl-3; le polyglycéryl-3-nonaisostearate; le polyglycéryl-3-nonanoate;
      - les esters partiels de polyglycérol-10 et en particulier le polyglycéryl-10 nonaisostearate; le polyglycéryl-10-nonanoate; le polyglycéryl-10-isostéarate, le polyglycéryl-10-diisostéarate, le triisostéarate de polyglycéryl-10;
      - les monoesters de propylène glycol comme le monoisostearate de propylène glycol, le néopentanoate de propylène glycol, le monooctanoate de propylène glycol;
      - les monoesters de dimères-diols comme l'isostéaryl dimer dilinoleate et l'octyl dodecyl dimer dilinoleate
      - les éthers de glycérol, tels que le polyglycéryl-2 oleyléther, le polyglycéryl-3 cétyléther, le polyglycéryl-3 décyltétradécyléther et le polyglycéryl-2 stéaryléther;
      - les esters entre acide mono-, di- ou tri-carboxylique hydroxylé et monoalcools, et en particulier:
      - les esters, notamment monoesters, d'acide 12-hydroxystéarique; tels que l'hydroxystéarate d'octyle, et l'octyl-2 dodecyl hydroxystearate; on peut également citer les polyhydroxystéarates oligomères correspondants, notamment ayant un degré de polymérisation de 1 à 10, possédant au moins un OH résiduel;
      - les esters d'acide lactique, et notamment les lactates d'alkyles en C4-40, tels que le lactate de 2-éthylhexyle, le lactate de diisostéaryle, le lactate d'isostéaryle, le lactate d'isononyle, le lactate d'octyl-2 dodécyle;
      - les esters d'acide malique, et notamment les malates d'alkyles en C4-40, tels que le malate de diéthyl-2 hexyle, le malate de diisostéaryle, le malate de dioctyl-2 dodécyle;
      - les esters d'acide citrique, et notamment les citrates d'alkyles en C4-40, tels que le citrate de triisostéaryle, le citrate de triisocétyle et le citrate de triisoarachidyle.
(iii) les huiles naturelles, naturelles modifiées, végétales, hydroxylées et notamment :
   - les esters triglycériques portant un ou plusieurs OH,
   - l'huile de ricin, hydrogénée ou non, ainsi que ses dérivés notamment issus de la transesterification de l'huile de ricin; comme les produits Polycin M-365 ou Polycin 2525 vendus par Vertellus;
   - les huiles époxydées modifiées, la modification consistant à ouvrir la fonction époxy pour obtenir un diol, et notamment l'huile de soja modifiée hydroxylée; les huiles de soja hydroxylées (directement hydroxylées ou d'abord époxydées); et notamment les huiles Agrol 2.0, Agrol 3.0 ou Agrol 7.0 commercialisées par Bio-Based Technologies, LLC; l'huile Soyol R2-052 de la société Urethane Soy System; les huiles Renuva commercialisées par Dow Chemical; les huiles BioH Polyol 210 et 500 commercialisées par Cargill.

En particulier, lorsque l'on utilise des huiles brillantes, on peut employer les huiles brillantes suivantes, pour lesquelles l'indice de réfraction à 25°C est indiqué entre parenthèses: le polyglycéryl-3-diisostéarate (1,472), le phytantriol (1,467), l'huile de ricin (1,475), l'octyl-2-dodécanol (1,46), l'alcool oléylique (1,461), l'hydroxystéarate d'octyle (1,46), le polyglyceryl-2-isostéarate (1,468), le polyglyceryl-2-diisostearate (1,464), le malate de diisostéaryle (1,462), le butyl-2 octanol, l'hexyl-2 décanol (1,45), le décyl-2 tétradécanol (1,457), ainsi que leurs mélanges.

De préférence, les huiles susceptibles d'être employées dans la présente invention sont choisies parmi l'octyl-2-dodécanol, le malate de diisostéaryle, le butyl-2 octanol, l'hexyl-2 décanol, le décyl-2 tétradécanol; l'huile de ricin hydrogénée ou non, ainsi que ses dérivés; l'huile de soja modifiée hydroxylée, et leurs mélanges.

Le groupe de jonction susceptible d'être utilisé pour former le composé selon l'invention porte au moins un groupe réactive, notamment isocyanate ou imidazole, susceptible de réagir avec les fonctions réactives, notamment OH et/ou NH₂ (NH₂ uniquement pour l'imidazole), de l'huile, afin de former une liaison covalente, notamment de type uréthanne, entre ladite huile et ledit groupe de jonction.

Ledit groupe de jonction est capable d'établir des liaisons H avec un ou plusieurs groupes de jonction partenaires, de nature chimique identique ou différente, chaque appariement d'un groupe de jonction faisant intervenir au moins 3 liaisons H (hydrogène), de préférence au moins 4 liaisons H, préférentiellement 4 liaisons H. Par "groupe de jonction", on entend au sens de l'invention, tout groupe fonctionnel comportant des groupes donneurs ou accepteurs de liaisons H, et capable d'établir au moins trois liaisons H, de préférence au moins 4 liaisons H, préférentiellement 4 liaisons H, avec un groupe de jonction partenaire, identique ou non. Par "groupe de jonction partenaire", on entend au sens de l'invention, tout groupe de jonction pouvant établir des liaisons H avec un ou plusieurs groupes de jonction d'un même ou d'un autre polymère selon l'invention. Les groupes de jonction peuvent être de nature chimique identique ou différente. S'ils sont identiques, ils peuvent alors établir des liaisons H entre eux et sont alors appelés groupes de jonction auto-complémentaires. S'ils sont différents, ils sont choisis de telle façon qu'ils soient complémentaires vis à vis des interactions H.

Ledit groupe de jonction, porteur de groupes isocyanates, peut donc être schématisé (G)-(NCO)ₚ, p étant un entier non nul, de préférence égal à 1 ou 2.

Le groupe de jonction comprend par ailleurs au moins un motif monovalent de formule (I) et/ou au moins un motif divalent de formule (II), telles que ci-dessous définies: 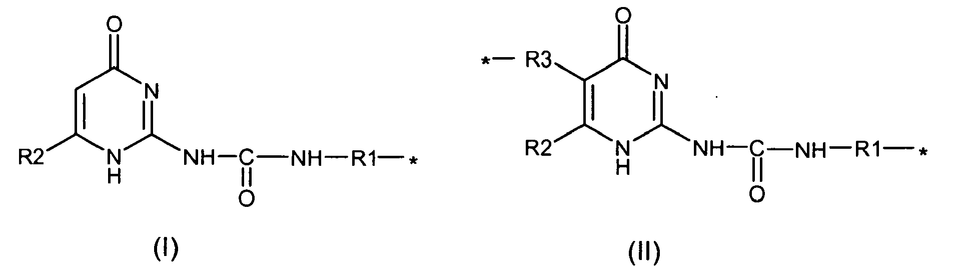 dans lesquelles :
- R1 et R3, identiques ou différents, représentent un radical carboné divalent choisi parmi (i) un groupe alkyle linéaire ou ramifié en C₁-C₃₂, (ii) un groupe cycloalkyle en C₄-C₁₆ et (iii) un groupe aryle en C₄-C₁₆, comprenant éventuellement 1 à 8 hétéroatomes choisis parmi O, N, S, F, Si et P; et/ou éventuellement substitué par une fonction ester, amide ou par un radical alkyle en C₁-C₁₂, ou un mélange de ces groupes;
- R2 représente un atome d'hydrogène ou un radical carboné, notamment hydrocarboné (alkyle), linéaire, ramifié ou cyclique, saturé ou insaturé, éventuellement aromatique, en C1-C32, pouvant comprendre un ou plusieurs hétéroatomes choisis parmi O, N, S, F, Si et P.

Notamment, le radical R1 peut notamment être :
- un groupe alkylène divalent, linéaire ou ramifié, en C2-C12, notamment un groupe 1,2-éthylène, 1,6-hexylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène).
- un groupe cycloalkylène ou arylène, divalent, en C4-C12, notamment choisi parmi les radicaux suivants -isophorone-, tolylène, 2-méthyl-1,3-phénylène, 4-méthyl-1,3-phénylène; 4,4'-méthylènebiscyclohexylène; 4,4-bisphénylèneméthylène; ou de structure :

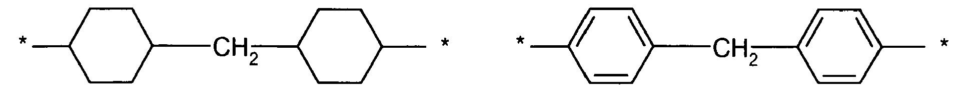

Par-isophorone-, on entend le radical divalent de structure :

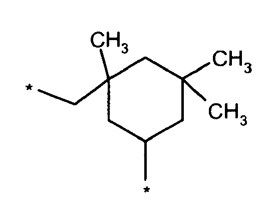

Préférentiellement, R1 représente -isophorone-, -(CH₂)₆- ou 4,4'-méthylènebiscyclohexylène.

Notamment, le radical R2 peut notamment être H, ou bien :
- un groupe alkyle en C₁-C₃₂, en particulier en C1-C16, voire en C1-C10;
- un groupe cycloalkyle en C₄-C₁₂ ;
- un groupe aryle en C₄-C₁₂ ;
- un groupe aryl(C₄-C₁₂) alkyle en C₁-C₁₈
- un groupe alcoxy en C₁-C₄ ;
- un groupe arylalcoxy, en particulier un groupe aryle (C₁-C₄) alcoxy ;
- un hétérocycle en C₄-C₁₂
ou une combinaison de ces radicaux, qui peuvent éventuellement être substitués par une fonction amino, ester et/ou hydroxy.

De préférence R2 représente H, CH₃, éthyle, C₁₃H₂₇, C₇H₁₅, phényle, isopropyle, isobutyle, n-butyle, tert-butyle, n-propyle, ou encore -CH(C₂H₅)(C₄H₉).

De préférence, R3 représente un radical divalent -R'3-O-C(O)-NH-R'4- dans lequel R'3 et R'4, identiques ou différents, représentent un radical carboné divalent choisi parmi un groupe alkyle linéaire ou ramifié en C₁-C₃₂ ou un groupe cycloalkyle en C₄-C₁₆ ou un groupe aryle en C₄-C₁₆, ou leur mélange.
En particulier, R'3 et R'4 peuvent représenter méthylène, 1,2-éthylène, 1,6-hexylène, 1,4-butylène, 1,6-(2,4,4-triméthylhexylène), 1,4-(4-méthylpentylène), 1,5-(5-méthylhexylène); 1,6-(6-méthylheptylène); 1,5-(2,2,5-triméthylhexylène), 1,7-(3,7-diméthyloctylène); 4,4'-méthylènebiscyclohexylène; 2-méthyl-1,3-phénylène; 4-méthyl-1,3-phénylène; 4,4'-bisphénylèneméthylène; 1,2-tolylène, 1,4-tolylène, 2,4-tolylène, 2,6-tolylène; 1,5-naphtylène; tétraméthylxylylène; isophorone.
Tout particulièrement, R'3 peut représenter un alkylène en C1-C4, notamment 1,2-éthylène.
De préférence, R'4 peut représenter le radical divalent dérivé de l'isophorone.

Tout particulièrement, R3 peut être de structure :

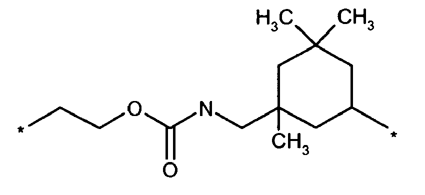

De façon particulièrement préférée, dans la formule (I), on peut avoir :
- R₁ = -isophorone-, R2 = méthyl, ce qui conduit au motif de formule : 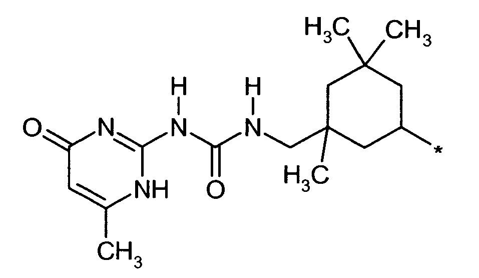
- R₁ = -(CH₂)₆-, R2 = méthyl, ce qui conduit au motif de formule : 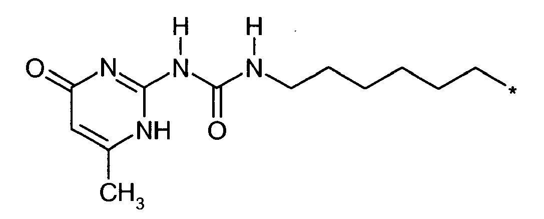
- R₁ = -(CH₂)₆-, R2 = isopropyl, ce qui conduit au motif de formule : 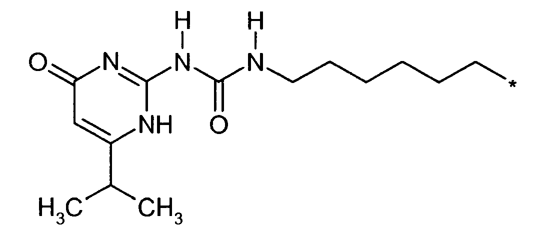
- R₁ = 4,4'-méthylènebiscyclohexylène et R2 = méthyle, ce qui conduit au motif de formule :

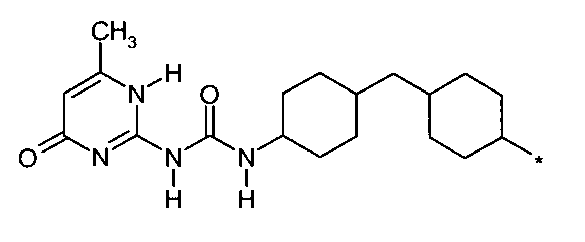

De façon particulièrement préférée, dans la formule (II), R1 représente le radical - isophorone-, R2= méthyle et R3=-(CH₂)₂OCO-NH-isophorone-, ce qui conduit au motif divalent de formule :

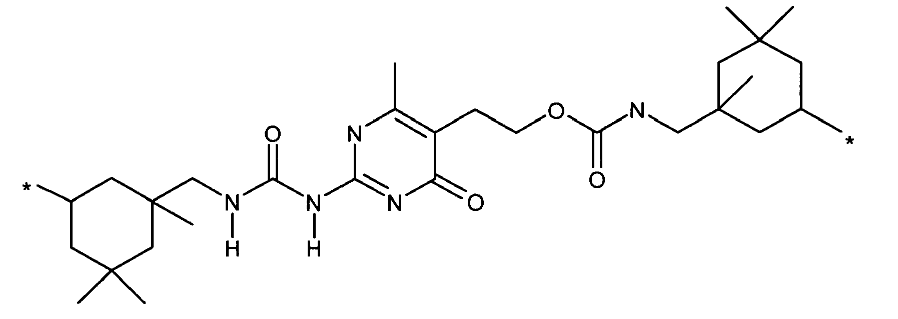

Les groupes de jonction porteurs d'une seule fonction isocyanate peuvent être de formule : 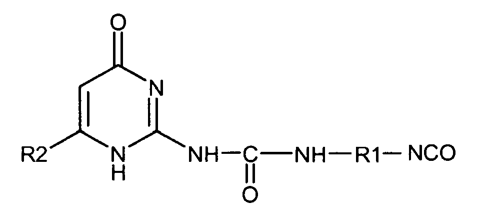 dans laquelle R1 et R2 sont tels que définis ci-dessus; et en particulier :
- R1 représente -isophorone-, -(CH₂)₆-, -CH₂CH(CH₃)-CH₂-C(CH₃)₂-CH₂-CH₂, 4,4'-méthylènebiscyclohexylène, 2-méthyl-1,3-phénylène; et/ou
- R2 représente H, CH₃, éthyle, C₁₃H₂₇, C₇H₁₅, phényle, isopropyle, isobutyle, n-butyle, tert-butyle, n-propyle, ou encore -CH(C₂H₅)(C₄H₉).

De manière préférée, les groupes de jonction peuvent être choisis parmi les groupes suivants :

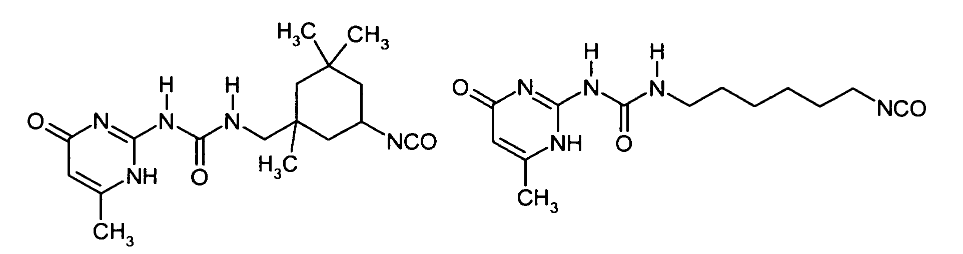

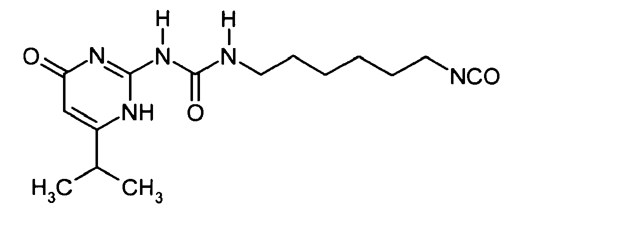

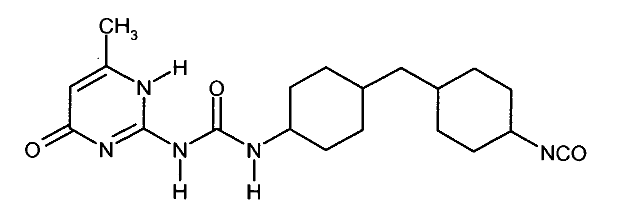

Les groupes de jonction porteurs de deux fonctions isocyanate peuvent être de formule : 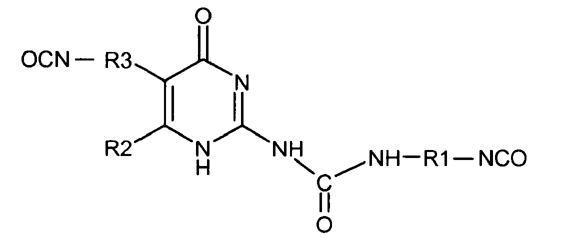 dans laquelle R1, R2 et R3 sont tels que définis ci-dessus, et en particulier :
- R1 représente -isophorone-, -(CH₂)₂-, -(CH₂)₆-, -CH₂CH(CH₃)-CH₂-C(CH₃)₂-CH₂-CH₂, 4,4'-méthylènebiscyclohexylène, 2-méthyl-1,3-phénylène; et/ou
- R2 représente H, CH₃, éthyle, C₁₃H₂₇, C₇H₁₅, phényle, isopropyle, isobutyle, n-butyle, tert-butyle, n-propyle, ou encore -CH(C₂H₅)(C₄H₉); et/ou
- R3 représente un radical divalent -R'3-O-C(O)-NH-R'4- dans lequel R'3 et R'4, identiques ou différents, représentent un radical carboné divalent choisi parmi un groupe alkyle linéaire ou ramifié en C₁-C₃₀ ou un groupe cycloalkyle en C₄-C₁₂ ou un groupe aryle en C₄-C₁₂; ou leur mélanges; et notamment R'3 représente un alkylène en C1-C4, notamment 1,2-éthylène et R'4 représente le radical divalent dérivé de l'isophorone.

Un groupe de jonction tout particulièrement préféré est celui de formule :

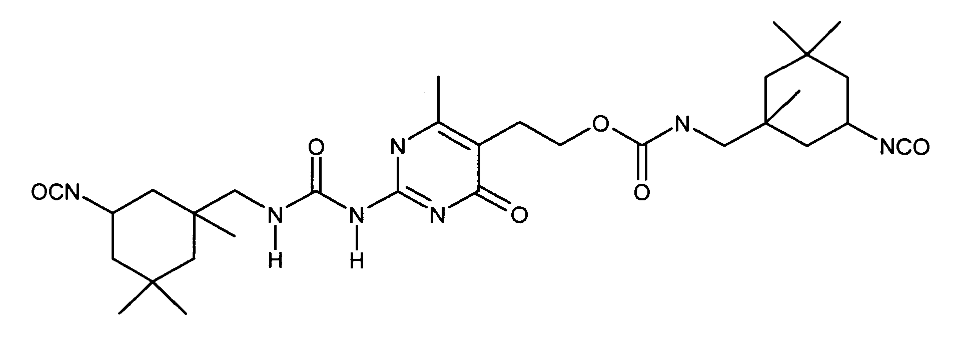

Parmi les groupes de jonction portant un groupe imidazole, on peut citer le composé suivant :

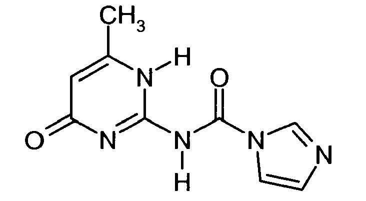

Selon un mode particulier de réalisation de l'invention, les groupes de jonction peuvent être fixés sur l'huile via la fonctionnalisation du groupe de jonction par un isocyanate ou imidazole.
Selon un autre mode de réalisation, il est possible de faire la réaction inverse en préfonctionnalisant l'huile par un diisocyanate.

Ainsi que mentionné ci-dessus (1^{er} mode), le composé selon l'invention peut donc résulter de la réaction chimique entre une huile (HB)-(OH)ₘ(NH₂)ₙ et un groupe de jonction (G)-(NCO)p ou (G)-(imidazole)ₚ.
De préférence, l'huile ne comprend que des fonctions hydroxyles et le groupe de jonction comprend 1 ou 2 fonctions isocyanate, ce qui conduit aux réactions suivantes :
(HB)-(OH)ₘ + OCN-(G)-NCO → (HB)-OC(O)NH-(G)-NHC(O)-(HB)
(HB)-(OH)ₘ + (G)-NCO → (HB)-OC(O)NH-(G)
avec m = entier supérieur ou égal à 1.

De préférence, le taux de greffage des OH libres de l'huile est compris entre 1 et 100%, notamment entre 20 et 99%, et mieux entre 50 et 95%; de préférence, ce taux est de 100% (la totalité des OH libres est fonctionnalisé par un groupe de jonction), notamment lorsque l'huile ne comporte initialement qu'un fonction OH.

Le composé selon l'invention peut être préparé par les procédés usuellement employés par l'homme du métier pour former une liaison uréthanne, entre les fonctions OH libres de l'huile et les fonctions isocyanates portées par le groupe de jonction. A titre d'illustration, un procédé général de préparation consiste à :
- s'assurer que l'huile à fonctionnaliser ne comporte pas d'eau résiduelle,
- chauffer l'huile comportant au moins une fonction réactive, notamment OH, à une température pouvant être comprise entre 60°C et 140°C;
- ajouter le groupe de jonction portant les fonctions réactives, notamment isocyanate;
- éventuellement agiter le mélange, sous atmosphère contrôlée, à une température de l'ordre de 100-130°C; pendant 1 à 24 heures;
- suivre par spectroscopie infrarouge, la disparition de la bande caractéristique des isocyanates (compris entre 2500 et 2800 cm-1) de manière à arrêter la réaction à la disparition totale du pic, puis à laisser revenir à température ambiante le produit final.

La réaction peut être effectuée en présence d'un solvant, notamment la méthyltétrahydrofurane, le tétrahydrofurane, le toluène ou l'acétate de butyle; la réaction peut aussi être effectuée sans solvant, l'huile pouvant alors servir de solvant.
Il est également possible d'ajouter un catalyseur conventionnel de la formation de liaison uréthane. A titre d'exemple, on peut citer le dilaurate dibutyle étain.
Le composé peut au final être lavé et séché, voire purifié, selon les connaissances générales de l'homme du métier.

Selon le 2^{ème} mode de réalisation, la réaction peut comporter les étapes suivantes:
(i) fonctionnalisation de l'huile par un diisocyanate selon le schéma réactionnel :
   (HB)-OH (1 éq.)+ NCO-X-NCO (1 éq.) → (HB)-OC(O)-NH-X-NCO
   puis
(iia) soit réaction avec la 6-méthylisocytosine : 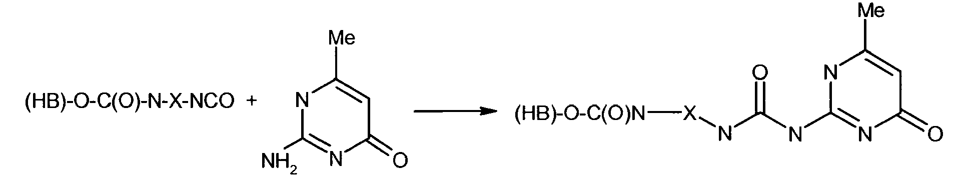 ou
(iib) soit réaction avec la 5-hydroxyéthyl-6-méthyl isocytosine :

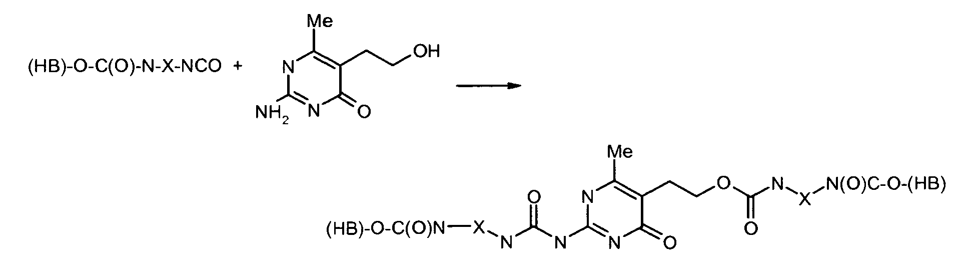

Une illustration d'une telle réaction est donnée dans FOLMER et al., Adv. Mater, 12, 874-78 (2000).

Les composés selon l'invention peuvent notamment répondre aux structures suivantes :
- l'octyldodécanol fonctionnalisé uréidopyrimidone de structure : 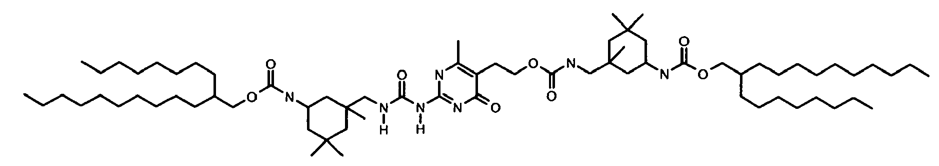 Ou bien de structure : 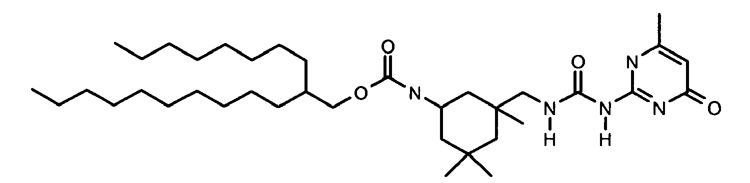
- le malate de diisostéaryle fonctionnalisé uréidopyrimidone de structure : 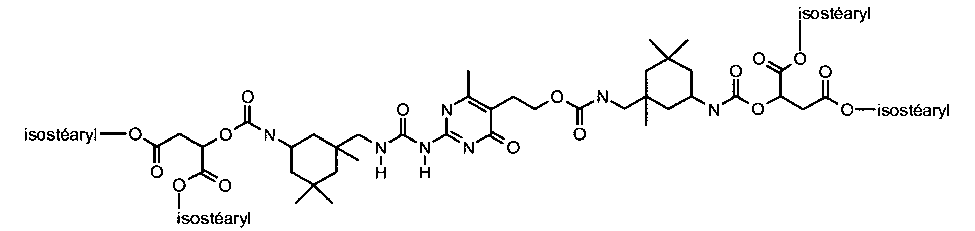 Ou bien de structure : 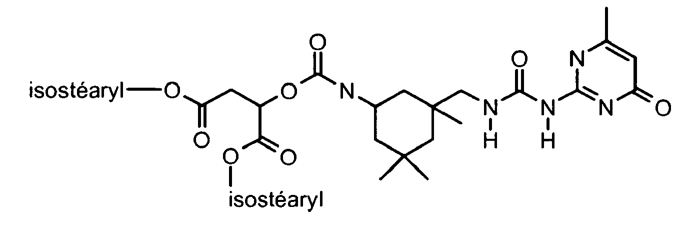
- l'huile de ricin fonctionnalisée uréidopyrimidone de structure : 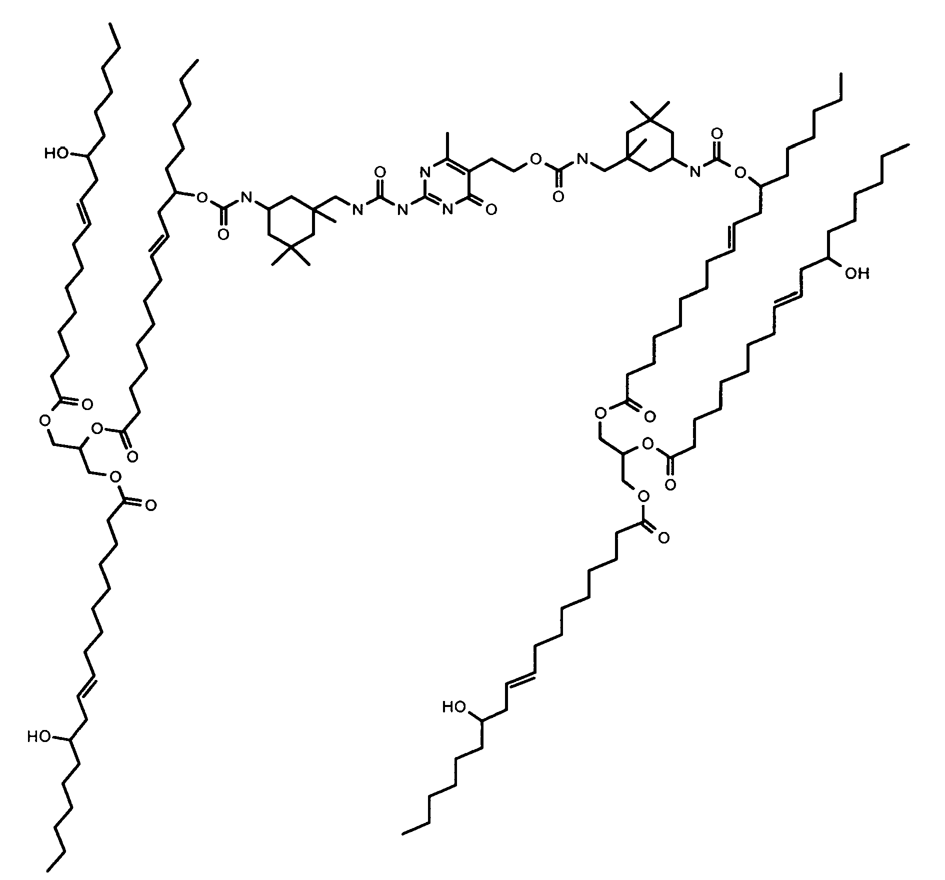 Ou bien de structure : 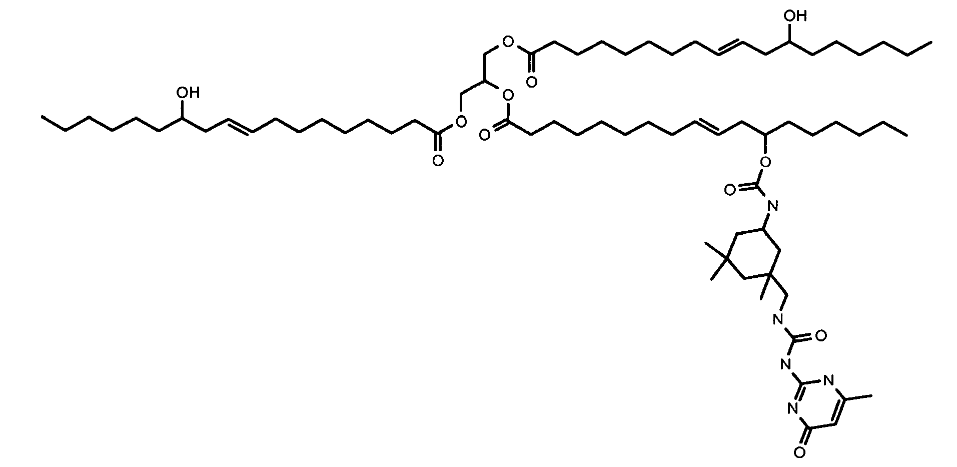
- l'hexyl-2 décanol fonctionnalisé uréidopyrimidone de structure : 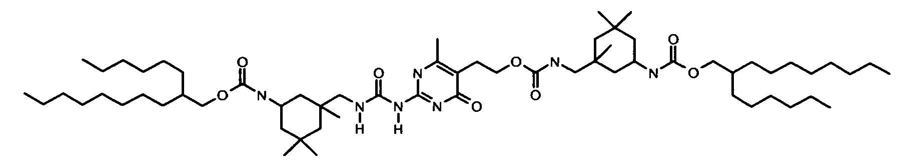 Ou bien de structure : 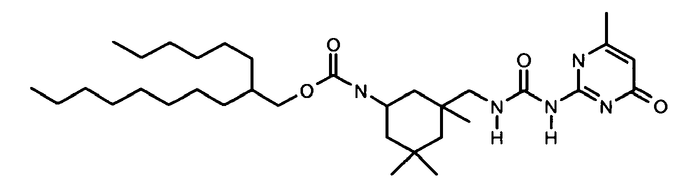
- le décyl-2 tétradécanol fonctionnalisé uréidopyrimidone de structure : 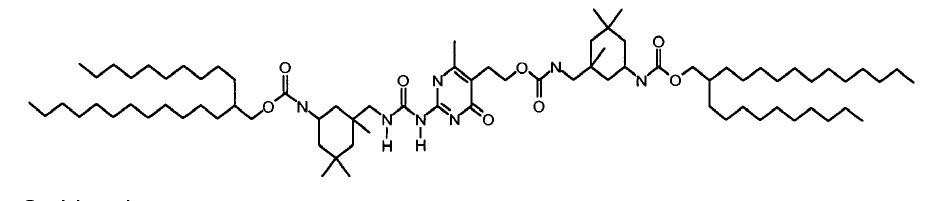 Ou bien de structure :

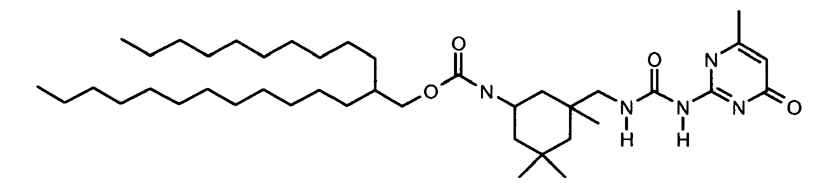

On a constaté que l'utilisation des composés selon l'invention peut conduire, après application de la composition sur les matières kératiniques, à la formation d'un polymère supramoléculaire sous forme de réseau réticulé physiquement, notamment à travers des liaisons hydrogène, se présentant généralement sous forme de film, et ayant une très bonne résistance mécanique.
Par "polymère supramoléculaire", on entend au sens de l'invention, une chaîne ou un réseau polymérique formé de l'assemblage de composés non polymères selon l'invention avec au moins un autre composé non polymère selon l'invention, identique ou différent, chaque assemblage comprenant au moins une paire de groupes de jonction appariés, identiques ou différents.
Par "paire de groupes de jonction appariés", on entend au sens de l'invention, deux groupes de jonction dont chacun peut être porté ou non par un même composé selon l'invention, les deux groupes étant reliés ensemble via 4 liaisons H. Ainsi le polymère supramoléculaire présentera des points de réticulation physique assurés par les liaisons H entre ces paires de groupes de jonction. La réticulation physique assurera le maintien et la persistance de l'effet cosmétique de manière analogue à la réticulation chimique, tout en permettant la réversibilité, c'est-à-dire la possibilité d'éliminer totalement le dépôt.

De préférence, le composé selon l'invention présente une viscosité, mesurée à 125°C, comprise entre 30 et 6000 mPa.s, notamment entre 150 et 4000 mPa.s, voire entre 500 et 3500 mPa.s et encore mieux entre 750 et 3000 mPa.s.

La masse moléculaire moyenne en nombre (Mn) du composé selon l'invention est de préférence comprise entre 180 à 8000, de préférence 200 à 6000, voire de 300 à 4000, et encore mieux de 400 à 3000, préférentiellement de 500 à 1500.

Le composé selon l'invention est avantageusement soluble dans les milieux huileux cosmétiques usuellement employés, et notamment dans les huiles végétales, les alcanes en C6-C32, les esters gras en C8-C32, les esters courts en C2-C7, les alcools gras en C8-C32, et plus particulièrement dans les milieux comprenant au moins de l'isododécane, du Parléam, de l'isononanoate d'isononyle, de l'octyldodécanol, du benzoate d'alkyle en C12-C15, de l'acétate de butyle, de l'acétate d'éthyle, seul ou en mélange.
Par soluble, on entend que le composé forme une solution limpide dans au moins un solvant choisi parmi l'isododécane, le Parléam, l'isononanoate d'isononyle, l'octyldodécanol, le benzoate d'alkyle en C12-C15, l'acétate de butyle, l'acétate d'éthyle, à raison d'au moins 50% en poids, à 25°C.

Les composés selon l'invention peuvent être utilisés avantageusement dans une composition cosmétique, qui comprend par ailleurs un milieu cosmétiquement ou dermatologiquement acceptable, c'est-à-dire compatible avec les matières kératiniques telles que la peau du visage ou du corps, les cils, les sourcils, les lèvres et les ongles.
La quantité de composé présent dans les compositions dépend bien entendu du type de composition et des propriétés recherchées et peut varier à l'intérieur d'une gamme très large, comprise généralement entre 5 et 80% en poids, de préférence entre 10 et 75% en poids, notamment entre 20 et 70% en poids, voire entre 25 et 65% en poids, et mieux entre 30 et 60% en poids, par rapport au poids de la composition cosmétique finale.

La composition peut alors comprendre, selon l'application envisagée, les constituants habituels à ce type de composition.
La composition selon l'invention peut avantageusement comprendre une phase grasse liquide, qui peut constituer un milieu solvant des polymères selon l'invention, et qui peut comprendre au moins un composé choisi parmi les huiles et/ou solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.
Par 'volatil', on entend au sens de l'invention, tout composé susceptible de s'évaporer au contact des matières kératiniques, ou des lèvres, en moins d'une heure, à température ambiante (25°C) et pression atmosphérique (1 atm). Notamment, ce composé volatil a une pression de vapeur non nulle, à température ambiante et pression atmosphérique, notamment allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par opposition, on entend par 'non volatil', un composé restant sur les matières kératiniques ou les lèvres à température ambiante et pression atmosphérique, au moins une heure et ayant notamment une pression de vapeur inférieure à 10⁻³ mm de Hg (0,13Pa).

De préférence, le milieu physiologiquement acceptable de la composition selon l'invention peut comprendre, dans une phase grasse liquide, au moins une huile et/ou un solvant qui peut être choisi parmi, seul ou en mélange :
1/ les esters des acides monocarboxyliques avec les monoalcools et polyalcools; avantageusement, ledit ester est un benzoate d'alkyle en C12-C15 ou répond à la formule suivante : R'₁-COO-R'₂ où :
   R'₁ représente un radical alkyle linéaire ou ramifié de 1 à 40 atomes de carbone, de préférence de 7 à 19 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles, et
   R'₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, éventuellement substitué et dont la chaîne hydrocarbonée peut être interrompue par un ou plusieurs hétéroatomes choisis parmi N et O et/ou une ou plusieurs fonctions carbonyles.
      Par "éventuellement substitué", on entend que R'₁ et/ou R'₂ peuvent porter un ou plusieurs substituants choisis, par exemple, parmi les groupements comprenant un ou plusieurs hétéroatomes choisi parmi O et/ou N, tels que amino, amine, alcoxy, hydroxyle.
      Des exemples des groupes R'₁ sont ceux dérivés des acides gras de préférence supérieur choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges. De préférence, R'₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone.
      On peut en particulier citer, de préférence, les esters en C₈-C₄₈, éventuellement incorporant dans leur chaîne hydrocarbonée un ou plusieurs hétéroatomes parmi N et O et/ou une ou plusieurs fonctions carbonyle; et plus particulièrement l'huile de purcellin (octanoate de cétostéaryle), l'isononanoate d'isononyle, le myristate d'isopropyle, le palmitate d'isopropyle, le palmitate d'éthyl-2-hexyle, le stéarate d'octyl-2-dodécyle, l'érucate d'octyl-2-dodécyle, l'isostéarate d'isostéaryle, le benzoate d'alcool en C₁₂ à C₁₅, le laurate d'hexyle, l'adipate de diisopropyle; et les heptanoates, octanoates, décanoates ou ricinoléates d'alcools ou de polyalcools, par exemple d'alcools gras comme le dioctanoate de propylène glycol, ainsi que le N-lauroyl sarcosinate d'isopropyle (notamment Eldew-205SL d'Ajinomoto); les esters hydroxylés comme le lactate d'isostéaryle, le malate de di-isostéaryle; et les esters du pentaérythritol; les esters ramifiés en C8-C16, notamment le néopentanoate d'isohexyle.
2/ les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat, de jojoba, de palme, de calophyllum; ou encore les triglycérides des acides caprylique/caprique comme ceux vendus par la société Stearinerie Dubois ou ceux vendus sous les dénominations "Miglyol 810®", "812® " et "818® " par la société Dynamit Nobel.
3/ les alcools, et notamment les monoalcools, en C6-C32, notamment C12-C26, comme l'alcool oléïque, l'alcool linoléique, l'alcool linolénique, l'alcool isostéarylique, le 2-hexyldécanol, le 2-butyloctanol, le 2-undécylpentadécanol et l'octyldodécanol;
4/ les huiles hydrocarbonées, linéaires ou ramifiés, volatiles ou non, d'origine synthétique ou minérale, qui peuvent être choisies parmi les huiles hydrocarbonées ayant de 5 à 100 atomes de carbones, et notamment la vaseline, les polydécènes, les polyisobutènes hydrogénés tel que le Parléam, le squalane, le perhydrosqualène et leurs mélanges.
   On peut plus particulièrement citer les alcanes linéaires, ramifiés et/ou cycliques en C5-C48, et préférentiellement les alcanes ramifiés en C8-C16 comme les isoalcanes en C8-C16 d'origine pétrolière (appelées aussi isoparaffines); notamment le décane, l'heptane, le dodécane, le cyclohexane; ainsi que l'isododécane, l'isodécane, l'isohexadécane.
5/ les huiles de silicone, volatiles ou non volatiles;
   Comme huiles de silicone volatiles, on peut citer les huiles de silicones linéaires ou cycliques volatiles, notamment celles ayant une viscosité inférieure à 8 centistokes, et ayant notamment de 2 à 10 atomes de silicium, ces silicones comportant éventuellement des groupes alkyles ou alkoxy ayant de 1 à 22 atomes de carbone; et en particulier l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'hexaméthyldisiloxane, l'octaméthyltrisiloxane, le décaméthyltétrasiloxane, le dodécaméthylpentasiloxane, le méthylhexyldiméthylsiloxane et leurs mélanges.
   Les huiles de silicone non volatiles utilisables selon l'invention peuvent être les polydiméthylsiloxanes (PDMS), les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, les 2-phényléthyl triméthylsiloxysilicates.

Préférentiellement, le milieu physiologiquement acceptable de la composition selon l'invention comprend, dans une phase grasse liquide, au moins une huile et/ou un solvant choisi parmi, seul ou en mélange, l'isododécane, le Parléam, l'isononanoate d'isononyle, l'octyldodécanol, la phényl triméthicone, les benzoates d'alkyle en C12-C15, les acétates de butyle et d'éthyle, et/ou la D5 (décaméthyl-cyclopentasiloxane).

La phase grasse liquide peut en outre comprendre des huiles et/ou solvants additionnels, qui peuvent être choisis parmi, seul ou en mélange :
- les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés;
- les huiles d'origine animale;
- les éthers en C₆ à C₄₀, notamment en C10-C40; les éthers de propylène glycol liquides à température ambiante tels que le monométhyléther de propylène glycol, l'acétate de monométhyléther de propylène glycol, le mono n-butyl éther de dipropylène glycol ;
- les acides gras en C₈-C₃₂, comme l'acide oléique, l'acide linoléique, l'acide linolénique et leurs mélanges.
- les huiles bifonctionnelles, comprenant deux fonctions choisies parmi ester et/ou amide et comprenant de 6 à 30 atomes de carbone, notamment 8 à 28 atomes de carbone, mieux de 10 à 24 carbones, et 4 hétéroatomes choisis parmi O et N; de préférence les fonctions amide et ester étant dans la chaîne;
- les cétones liquides à température ambiante (25°C) tels que la méthyléthylcétone, la méthylisobutylcétone, la diisobutylcétone, l'isophorone, la cyclohexanone, l'acétone ;
- les aldéhydes liquides à température ambiante tels que le benzaldéhyde, l'acétaldéhyde;

La phase grasse liquide peut représenter 1 à 90% en poids de la composition, notamment de 5 à 75% en poids, en particulier de 10 à 60% en poids, voire de 25 à 55% en poids, du poids total de la composition.

La composition selon l'invention peut comprendre avantageusement un agent épaississant qui peut en particulier être choisi parmi :
- les silices notamment hydrophobes, telles que celles décrites dans le document EP-A-898960, et par exemple commercialisées sous les références "AEROSIL R812®" par la société Degussa, "CAB-O-SIL TS-530®", "CAB-O-SIL TS-610®", "CAB-O-SIL TS-720®" par la société Cabot, "AEROSIL R972®", "AEROSIL R974®" par la société Degussa ;
- les argiles telles que la montmorillonite, les argiles modifiées telles que les bentones par exemple, l'hectorite stéaralkonium, la bentonite stéaralkonium,
- les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

La quantité d'agent épaississant dans la composition selon l'invention peut aller de 0,05 à 40% en poids, par rapport au poids total de la composition, de préférence de 0,5 à 20% et mieux de 1 à 15% en poids.

La composition selon l'invention peut également comprendre au moins une cire d'origine végétale, animale, minérale ou de synthèse, voire siliconée.
On peut en particulier citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.
La quantité de cire dans la composition selon l'invention peut aller de 0,1 à 70% en poids, par rapport au poids total de la composition, de préférence de 1 à 40% en poids, et mieux de 5 à 30% en poids.

La composition selon l'invention peut également comprendre une ou plusieurs matières colorantes choisies parmi les composés pulvérulents comme les pigments, les charges, les nacres et les paillettes, et/ou les colorants liposolubles ou hydrosolubles.

Les matières colorantes, notamment pulvérulentes, peuvent être présentes, dans la composition, en une teneur de 0,01 à 50% en poids, par rapport au poids de la composition, de préférence de 0,1 à 40% en poids, voire de 1 à 30% en poids. Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.
Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium. Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la composition et mieux de 0,1 à 6 %.
Les colorants hydrosolubles sont par exemple le jus de betterave, le bleu de méthylène et peuvent représenter 0,01 à 6% du poids total de la composition.

La composition peut comprendre, en outre, d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les vitamines, les céramides, les filtres solaires, les tensioactifs, les gélifiants, les agents d'étalement, les agents mouillants, les agents dispersants, les anti-mousses, les neutralisants, les stabilisants, les polymères et notamment les polymères filmogènes liposolubles, et leurs mélanges. Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition cosmétique. Elles peuvent donc se présenter sous la forme d'une suspension, une dispersion notamment d'huile dans de l'eau grâce à des vésicules; une solution organique ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté. L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions conformes à l'invention présentant une brillance et une tenue de ladite brillance améliorées par rapport à l'état de l'art, elles peuvent être utilisées pour le soin ou le maquillage des matières kératiniques telles que la peau, les cils, les sourcils, les ongles, les lèvres, et plus particulièrement pour le maquillage des lèvres, des cils et/ou du visage.
Elles peuvent donc se présenter sous la forme d'un produit de soin et/ou de maquillage de la peau du corps ou du visage, des lèvres, des cils, des sourcils, ou des ongles; d'un produit solaire ou autobronzant; elles se présentent avantageusement sous forme de composition de maquillage, notamment de mascara, d'eyeliner, de rouge à lèvres, de brillant à lèvres (gloss), de fard à joues ou à paupières, de fond de teint, de vernis à ongles ou de soin des ongles.

L'invention a encore pour objet un procédé de traitement cosmétique des matières kératiniques, notamment de la peau du corps ou du visage, des lèvres, des ongles et/ou des cils, comprenant l'application sur lesdites matières d'une composition cosmétique telle que définie précédemment.
Ce procédé selon l'invention permet notamment le soin ou le maquillage desdites matières kératiniques, en particulier des lèvres et/ou des ongles, par application d'une composition notamment de rouge à lèvres, de brillant à lèvres (gloss), de soin des ongles ou de vernis à ongles selon l'invention.

L'invention est illustrée plus en détail dans les exemples de réalisation suivants.

### Exemple 1 : Octyldodécanol fonctionnalisé uréidopyrimidone

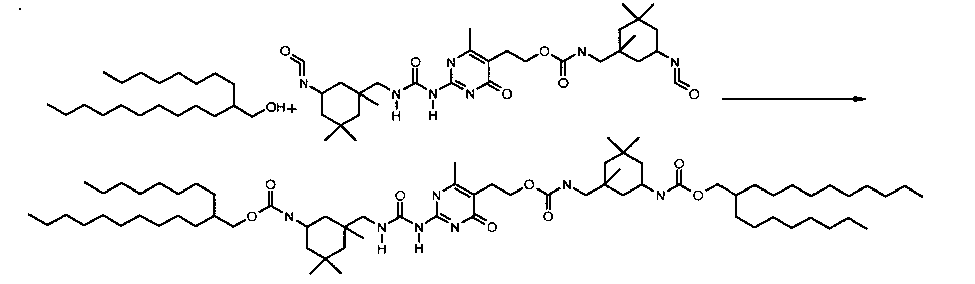

On met en solution 70 g d'uréidopyrimidone diisocyanate dans la méthyl tétrahydrofurane, sous argon. On ajoute 80,3 g d'octyldodécanol dans 100 ml de dichlorométhane, sous argon, puis 15 microlitre de dilaurate dibutyl étain (catalyseur). Le mélange réactionnel est chauffé à reflux jusqu'à disparition du pic de l'isocyanate (2250-2265 cm-1) en spectrométrie IR.
L'octyldodécanol en excès est éliminé par lavage successif du milieu réactionnel avec du méthanol, suivi de trois extractions et séchage sur MgSO₄. Après évaporation de la phase organique, on obtient 103 g d'une poudre légèrement jaune, caractérisée par RMN 1H (structure conforme).

On peut véhiculer cette poudre dans l'isododécane, par exemple à une concentration de 10% en poids; cette concentration peut aller notamment jusqu'à 60% en poids dans l'isododécane, qui conduit alors à une solution visqueuse mais toujours manipulable. On constate donc que par fonctionnalisation par une uréidopyrimidone, on passe d'une huile liquide à un solide, véhiculable dans l'isododécane à des concentrations supérieures à 30%.

Lorsque l'on applique une solution comprenant 50% en poids de composé dans l'isododécane, après évaporation du solvant, on obtient un film transparent et brillant, qui présente une bonne adhérence par fragmentation, et une faible résistance aux frottements.

### Exemple 2 : Malate de diisostéaryle fonctionnalisé par une uréidopyrimidone

On sèche sous pression réduite 15 g (0.0234 mole) de malate de diisostéaryle, à 80°C pendant 4 heures. On ajoute 7,21 g (0.0117 mole) d'uréidopyrimidone diisocyanate en solution dans 60 ml de méthyltétrahydrofurane, et 12 µl de catalyseur dilaurate dibutyle d'étain. Le mélange est chauffé à 95°C, sous argon, pendant 26 heures (disparition de la bande caractéristique des isocyanates par spectroscopie IR). On ajoute 20 ml de méthyltétrahydrofurane au mélange réactionnel, puis on filtre sur célite. Après évaporation du solvant et séchage sous pression réduite, on obtient un solide jaune pâle.

### Exemple 3 : Huile de ricin fonctionnalisée par une uréidopyrimidone

On sèche sous pression réduite 15 g d'huile de ricin (0.016 mole), à 80°C pendant 4 heures. On ajoute une solution de 4,9 g d'uréidopyrimidone diisocyanate (0.008 mole) dans 60 ml de méthyltétrahydrofurane, et 12 µl de catalyseur dilaurate dibutyle d'étain. Le mélange est chauffé à 90°C pendant 19 heures (disparition complète de la bande caractéristique des isocyanate par spectroscopie IR). A la fin de la réaction, le solvant est évaporé et le produit résultant est séché sous pression réduite, à 35°C pendant une nuit.
On obtient une gomme solide jaune pâle.

### Exemple 4 (comparatif à l'exemple 1): Octyldodécanol fonctionnalisé par l'isophorone

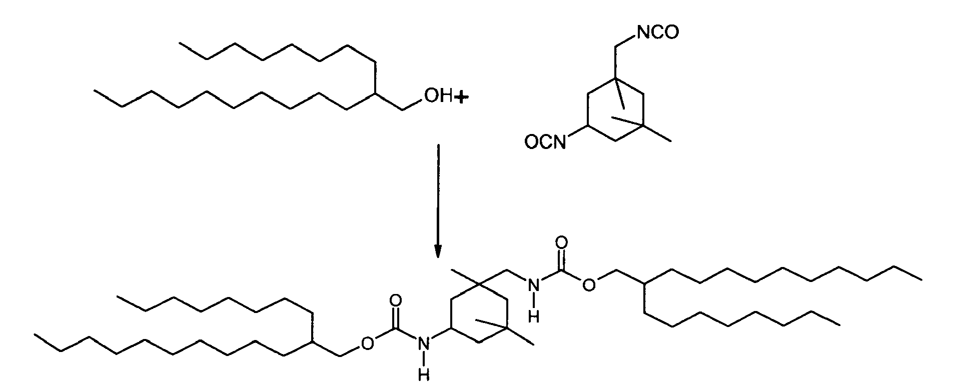

On sèche sous pression réduite 10 g d'octyldodécanol, à 80°C pendant 2 heures, puis on ajoute 3,72 g d'isophorone diisocyanate et 25 microlitre de catalyseur dilaurate dibutyl étain. Le mélange est chauffé à 95°C, sous argon. La disparition de l'isocyanate est suivie par spectroscopie IR (disparition de la bande entre 2250 et 2265cm⁻¹, après 12 heures de chauffage).
On obtient une huile visqueuse, ne formant pas un matériau cohésif.

### Exemple 5 (comparatif à l'exemple 2): Malate de dilsostéaryle fonctionnalisé par l'isophorone

On sèche sous pression réduite 10 g (0.0159 mole) de malate de diisostéaryle, à 80°C pendant 3 heures. On ajoute sous argon 1,77 g (0.079 moles) d'isophorone diisocyanate et 2,5 µl de catalyseur (dilaurate dibutyle d'étain), et le mélange réactionnel est chauffé à 95°C pendant 16 heures. Au cours de la réaction, la viscosité du milieu réactionnel augmente. La réaction est arrêtée après disparition du pic caractéristique des isocyanates par spectroscopie IR.

### Exemple 6 (comparatif à l'exemple 3): Huile de ricin fonctionnalisée par l'isophorone

On sèche sous pression réduite 15 g (0.016 mole) d'huile de ricin, à 80°C pendant 6 heures. On ajoute 1,78 g(0.008 mole) d'isophorone diisocyanate et 12 µl de catalyseur dilaurate dibutyle d'étain, et le mélange est chauffé à 90°C pendant 16 heures. La réaction est arrêtée après disparition du pic caractéristique des isocyanates par spectroscopie IR.

### Exemple 7

Les composés préparés aux exemples 1 à 6 sont observés, visuellement et au toucher, et les résultats sont récapitulés dans le tableau suivant :

| | Aspect physique du composé | Aspect du film * Indice de réfraction** (Indice réfraction huile non fonctionnalisée) |
|---|---|---|
| Exemple 1 | Solide jaune | Film brillant et collant, qui ne démouille pas; dépôt homogène. Sans transfert au doigt. 1,488 (1,46) |
| Exemple 4 (comparatif) | Huile visqueuse transparente | Film qui démouille; dépôt non homogène. Transfert au doigt. 1,474 (1,46) |
| Exemple 2 | Solide jaune | Film brillant peu collant, qui ne démouille pas; dépôt homogène. Sans transfert au doigt. 1,478 (1,462) |
| Exemple 5 (comparatif) | Huile visqueuse transparente | Film brillant collant qui démouille; dépôt non homogène. Sans transfert au doigt. 1,4598 (1,462) |
| Exemple 3 | Solide jaune (gomme solide) | Film brillant légèrement collant; comportement d'un solide fragile, qui ne démouille pas; dépôt homogène. Sans transfert au doigt. 1,4852 (1,48) |
| Exemple 6 (comparatif) | Huile visqueuse transparente | Film brillant très collant, qui démouille; dépôt non homogène. Transfert au doigt. 1,4813 (1,48) |

| | | |
|---|---|---|
| * Les films sont formés à partir d'une solution à 40% en poids du composé, soit dans l'isododécane pour les exemples 1-2 et 4-5, soit dans le tétrahydrofurane pour les exemples 3 et 6. ** Pour les mesures d'indices de réfraction, tous les films sont formés à partir d'une solution à 40% en poids du composé dans le tétrahydrofurane; l'indice de réfraction est mesuré après évaporation du solvant. | | |

Le film qui ne démouille pas signifie qu'après dépôt et évaporation du solvant, on obtint un 'vrai' film continu homogène.
Le film démouille signifie qu'après dépôt et évaporation du solvant, on obtient un film 'à trous', non homogène, non continu.

On effectue sur ces dépôts/films un test au tribomètre : les films sont formés à partir d'une solution à 40% en poids dans le tétrahydrofurane, par dépôt sur un élastomère nitrile, puis séchage pendant 24 heures à 25°C.
Les essais sont réalisés à l'aide d'un tribomètre CSEM muni d'une bille de diamètre 6mm. Cette bille soumise à une charge de 0,15N frotte de manière répétée sur un film (de 10 à 20 µm d'épaisseur). La vitesse de rotation du disque est fixée à 6,3 cm/s ce qui correspond à une fréquence d'un tour par seconde. L'essai est terminé lorsque l'usure est totale, ou bien stoppé après 1000 tours de sollicitation.

| | Observations |
|---|---|
| Exemple 1 | Le film reste inchangé (homogène) pendant 300 tours (pas d'usure ni de casse); le matériau est donc cohésif; comportement d'un solide. |
| Exemple 4 (comparatif) | Pas de mesure possible : le matériau est sans cohésion, il se comporte comme une huile |
| Exemple 2 | Le film reste inchangé (homogène) pendant 1000 tours (pas d'usure ni de casse); le matériau est donc très cohésif et ne s'use pas. |
| Exemple 5 (comparatif) | Le matériau se comporte comme une huile, avec un effet de beurrage quand il est soumis au test d'usure. |
| Exemple 3 | Le film est un peu cassant mais reste inchangé pendant 10 tours; après 10 tours, l'usure est plus nette; ceci traduit le comportement d'un solide |
| Exemple 6 (comparatif) | Pas de mesure possible car pas de film formé ab initio : comportement d'une huile |

On constate donc qu'il n'y a pas de diminution de l'indice de réfraction après fonctionnalisation. L'huile garde son caractère brillant, même fonctionnalisée. On constate également que la fonctionnalisation par des ureïdopyrimidones conduit à des films plus ou moins collants, mais qui ne transfèrent pas au doigt, contrairement aux films comparatifs.
De plus, et principalement, dans le cas des huiles fonctionnalisés par de l'isophorone (comparatives), les films démouillent et ne forment pas un dépôt homogène. A l'opposé, les films obtenus à partir des composés selon l'invention ne démouillent pas et sont homogènes et cohésifs. Les résultats au tribomètre confirment les propriétés de cohésion obtenues avec les composés de l'invention La fonctionnalisation par des ureïdopyrimidones conduit donc à des matériaux suffisamment cohésifs pour pouvoir assurer une rémanence du dépôt, par ailleurs brillant, supérieure à l'état de l'art (isophorone).
En résumé: la brillance est constante, le cohésif du dépôt amélioré et donc sa tenue améliorée.

### Exemple 8 : 2-hexyl décanol fonctionnalisé uréidopyrimidone

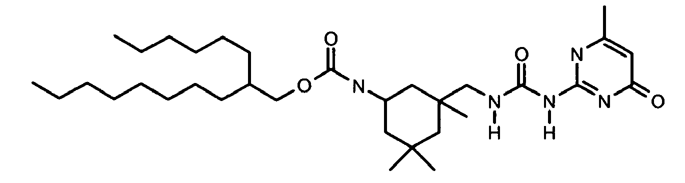

On chauffe 126,4 g de 2-hexyl décanol à 60°C sous pression réduite pendant 2 heures pour le sécher. Après 2 heures, l'huile est laissée revenir à 20°C sous argon, puis additionnée lentement, en 5 heures, à un mélange de 116 g d'isophorone diisocyanate et 55 mg de catalyseur DBTL à 50°C. A la fin de l'addition, la température du mélange réactionnel est portée à 110°C, puis on ajoute 90 ml de propylène carbonate, et 78,4 g de 6-méthyl isocytosine, ce qui conduit à une suspension blanche et homogène. L'agitation est maintenue à 110°C pendant deux heures et la disparition de l'isocyanate est suivie par spectroscopie Infrarouge. On observe la disparition du pic à 2250cm⁻¹. En parallèle, la disparition de l'amine provenant de l'isocytosine est suivie par un dosage d'amines. A la fin de la réaction, on ajoute 500 g d'isododécane, à 100°C, et on obtient une solution jaune pâle légèrement turbide. On ajoute 300 ml d'éthanol et l'agitation est maintenue pendant 2 heures. Après filtration sur célite, le mélange réactionnel est strippé à l'isododécane à 80°C afin d'éliminer l'alcool et le propylène carbonate. On obtient au final le produit désiré véhiculé dans l'isododécane à 50% d'extrait sec. Le produit est notamment caractérisé par HPLC et GPC (structure confirmée).

### Exemple 9 : 2-hexyl décanol fonctionnalisé uréidopyrimidone

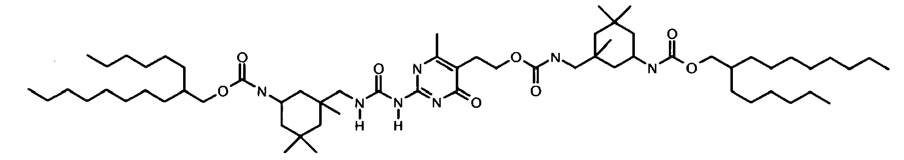

On chauffe 173,1 g de 2-hexyl décanol à 60°C sous pression réduite pendant 2 heures pour le sécher. Après 2 heures, l'huile est laissée revenir à 50°C sous argon, puis additionnée lentement, en 5 heures, à un mélange de 158,7 g d'isophorone diisocyanate et 77 mg de catalyseur DBTL à 50°C. A la fin de l'addition, la température du mélange réactionnel est portée à 110°C, puis on ajoute 150 ml de propylène carbonate, et 60,3 g de 5-hydroxyéthyl-6-méthyl isocytosine, ce qui conduit à une suspension blanche et homogène. L'agitation est maintenue à 110°C pendant cinq heures et la disparition de l'isocyanate est suivie par spectroscopie Infrarouge. On observe la disparition du pic à 2250cm⁻¹. A la fin de la réaction, on diminue la température du milieu réactionnel à 100°C, et on ajoute 780 g d'isododécane; on obtient un mélange turbide légèrement jaune. On ajoute 100 ml d'éthanol et l'agitation est maintenue pendant 2 heures. Après filtration sur célite, le mélange réactionnel est strippé à l'isododécane à 80°C afin d'éliminer l'alcool et le propylène carbonate.
On obtient au final le produit désiré véhiculé dans l'isododécane à 50% d'extrait sec. Le produit est notamment caractérisé par HPLC et GPC (structure confirmée).

### Exemple 10 : 2-décyl tétradécanol fonctionnalisé uréidopyrimidone

On chauffe 126 g de 2-décyl tétradécanol à 100°C sous pression réduite pendant 4 heures pour le sécher. Après 2 heures, l'huile est additionnée, en 4 heures, à 50°C et sous argon, à un mélange de 94,7 g d'isophorone diisocyanate et de catalyseur DBTL (qs). Un suivi par dosage d'isocyanate permet de suivre la réaction; à la demi-équivalence, on ajoute 126 g de propylène carbonate et 53,3 g de 6-méthyl isocytosine. L'agitation et le chauffage sont maintenus à 100°C pendant 16 heures et la disparition de l'isocyanate est suivie par spectroscopie Infrarouge. On observe la disparition du pic à 2250cm⁻¹. En parallèle, la disparition de l'amine provenant de l'isocytosine est suivie par un dosage d'amines. A la fin de la réaction, on ramène la température à 50°C, on ajoute 100 ml d'éthanol et on maintient l'agitation pendant 5 h. Après filtration sur célite et stripping à l'isododécane, on obtient le produit désiré véhiculé dans l'isododécane à 50% d'extrait sec. Le produit est notamment caractérisé par GPC et HPLC couplée à un spectre de masse.

### Exemple 11

On prépare un gloss comprenant (% en poids):
- 36% de composé préparé à l'exemple 1 (matière sèche)
- 5% de pigment DC Red 7.
- qsp 100% d'isododécane

Après application sur lèvre on forme un dépôt très brillant.
On prépare des gloss similaires avec les composés des exemples 2, 3, 8, 9 et 10.

### Exemple 12

On prépare un gloss comprenant :
- 50% de composé préparé à l'exemple 2 (matière sèche)
- 5% de pigment DC Red 7.
- qsp 100% d'isododécane
On prépare des gloss similaires avec les composés des exemples 1, 3, 8, 9 et 10.

### Exemple 13

On prépare un vernis à ongles comprenant (% en poids):
- Nitrocellulose 15 %
- polymère de l'exemple 3 (matière sèche) 9 %
- Acétyl citrate de tributyle 5 %
- pigments 1 %
- Hectorite 1,2 %
- Alcool Isopropylique 8%
- Acétate d'éthyle, acétate de butyle qsp 100%
On prépare des vernis similaires avec les composés des exemples 1, 2, 8, 9 et 10.

## Revendications

1. Composition cosmétique comprenant, dans un milieu cosmétiquement acceptable, un composé susceptible d'être obtenu par réaction entre :
- une huile portant au moins une fonction réactive nucléophile et/ou électrophile, et
- un groupe de jonction capable d'établir des liaisons hydrogène avec un ou plusieurs groupes de jonction partenaires, chaque appariement d'un groupe de jonction faisant intervenir au moins 3 liaisons hydrogène, ledit groupe de jonction portant au moins une fonction réactive susceptible de réagir avec la fonction réactive portée par l'huile, ledit groupe de jonction comprenant au moins en outre un motif de formule (I) ou (II): 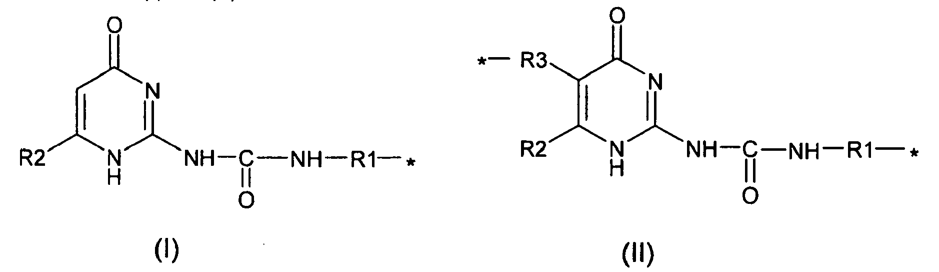 dans lesquelles :
- R1 et R3, identiques ou différents, représentent un radical carboné divalent choisi parmi (i) un groupe alkyle linéaire ou ramifié en C₁-C₃₂, (ii) un groupe cycloalkyle en C₄-C₁₆ et (iii) un groupe aryle en C₄-C₁₆; comprenant éventuellement 1 à 8 hétéroatomes choisis parmi O, N, S, F, Si et P; et/ou éventuellement substitué par une fonction ester, amide ou par un radical alkyle en C₁-C₁₂; ou un mélange de ces groupes;
- R2 représente un atome d'hydrogène ou un radical carboné, linéaire, ramifié ou cyclique, saturé ou insaturé, éventuellement aromatique, en C1-C32, pouvant comprendre un ou plusieurs hétéroatomes choisis parmi O, N, S, F, Si et P.

2. Composition selon la revendication 1, dans laquelle l'huile portant la fonction réactive est choisi parmi, seule ou en mélange :
(i) les alcools gras, comprenant 6 à 50 atomes de carbone, linéaires, ramifiés ou cycliques, saturés ou insaturés, comprenant 1 ou plusieurs OH; éventuellement comprenant un ou plusieurs NH₂;
(ii) les esters et les éthers portant au moins un groupe OH libre;
(iii) les huiles naturelles, naturelles modifiées, végétales, hydroxylées.

3. Composition selon l'une des revendications précédentes, dans laquelle l'huile portant la fonction réactive est choisi parmi, seule ou en mélange :
- les monoalcools linéaires ou ramifiés en C6-C50, saturés ou insaturés;
- les diols linéaires ou ramifiés en C6-C50, saturés ou insaturés;
- les triols linéaires ou ramifiés en C6-C50, saturés ou insaturés;
- les esters partiels de pentaérythritol,
- les diesters, triesters, tetraesters ou pentaesters de dipentaérythritol;
- les mono et di-esters de triméthylolpropane;
- les mono-, di- et tri-esters de di-triméthylolpropane;
- les mono-esters ou poly-esters partiels de glycérol ou de polyglycérols,
- les monoesters de propylène glycol ;
- les monoesters de dimères-diols ;
- les éthers de glycérol;
- les esters entre acide mono-, di- ou tri-carboxylique hydroxylé et monoalcools,
- les esters triglycériques portant un ou plusieurs OH,
- l'huile de ricin, hydrogénée ou non, ainsi que ses dérivés;
- les huiles époxydées modifiées, la modification consistant à ouvrir la fonction époxy pour obtenir un diol; les huiles de soja hydroxylées (directement hydroxylées ou d'abord époxydées).

4. Composition selon l'une des revendications précédentes, dans laquelle l'huile est une huile brillante, c'est-à-dire ayant un indice de réfraction supérieur ou égal à 1,46 à 25°C.

5. Composition selon l'une des revendications précédentes, dans laquelle l'huile a une masse molaire (Mw) comprise entre 150 et 6000 g/mol.

6. Composition selon l'une des revendications précédentes, dans laquelle l'huile est choisie parmi l'octyl-2-dodécanol, le malate de diisostéaryle, le butyl-2 octanol, l'hexyl-2 décanol, le décyl-2 tétradécanol; l'huile de ricin hydrogénée ou non, ainsi que ses dérivés; l'huile de soja modifiée hydroxylée; le polyglycéryl-3-diisostéarate, le phytantriol, l'alcool oléylique, l'hydroxystéarate d'octyle, le polyglyceryl-2-isostéarate, le polyglyceryl-2-diisostearate et leurs mélanges.

7. Composition selon l'une des revendications précédentes, dans laquelle, dans le groupe de jonction, le radical R1 représente :
- un groupe alkylène divalent, linéaire ou ramifié, en C2-C12, notamment un groupe 1,2-éthylène, 1,6-hexylène, 1,4-butylène, 1,6-(2,4,4-triméthylhexylène), 1,4-(4-méthylpentylène), 1,5-(5-méthylhexylène), 1,6-(6-méthylheptylène), 1,5-(2,2,5-triméthylhexylène), 1,7-(3,7-diméthyloctylène).
- un groupe cycloalkylène ou arylène, divalent, en C4-C12, notamment choisi parmi les radicaux suivants -isophorone-, tolylène, 2-méthyl-1,3-phénylène, 4-méthyl-1,3-phénylène; 4,4'-méthylènebiscyclohexylène; 4,4-bisphénylèneméthylène; ou de structure :

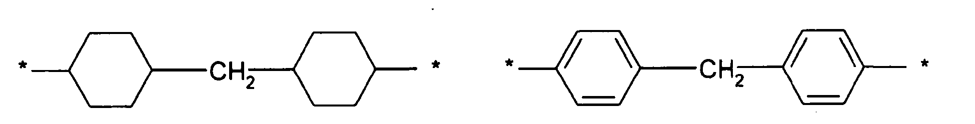

8. Composition selon l'une des revendications précédentes, dans laquelle, dans le groupe de jonction, le radical R2 représente H, ou bien :
- un groupe alkyle en C₁-C₃₂ ;
- un groupe cycloalkyle en C₄-C₁₂ ;
- un groupe aryle en C₄-C₁₂ ;
- un groupe aryl(C₄-C₁₂) alkyle en C₁-C₁₈
- un groupe alcoxy en C₁-C₄ ;
- un groupe aryle (C₁-C₄) alcoxy ;
- un hétérocycle en C₄-C₁₂
ou une combinaison de ces radicaux, qui peuvent éventuellement être substitués par une fonction amino, ester et/ou hydroxy.

9. Composition selon l'une des revendications précédentes, dans laquelle, dans le groupe de jonction, le radical R3 représente un radical divalent -R'3-O-C(O)-NH-R'4- dans lequel R'3 et R'4, identiques ou différents, représentent un radical carboné divalent choisi parmi un groupe alkyle linéaire ou ramifié en C₁-C₃₂ ou un groupe cycloalkyle en C₄-C₁₆ ou un groupe aryle en C₄-C₁₆ ; ou leur mélange.

10. Composition selon l'une des revendications précédentes, dans laquelle, dans le groupe de jonction,
(a) dans la formule (I), on a :
- R₁ = -isophorone- et R2 = méthyl,
- R₁ = -(CH₂)₆- et R2 = méthyl,
- R₁ = -(CH₂)₆- et R2 = isopropyl, ou
- R₁ = 4,4'-méthylènebiscyclohexylène et R2 = méthyle,
ou bien
(b) dans la formule (II), R1 représente le radical -isophorone-, R2= méthyle et R3=-(CH₂)₂OCO-NH-isophorone-.

11. Composition selon l'une des revendications précédentes, dans laquelle le groupe de jonction est de formule : 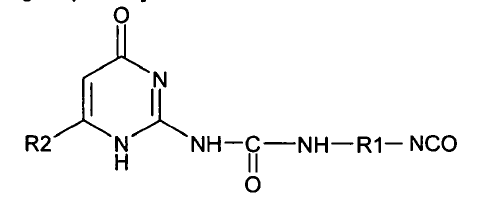 ou de formule : 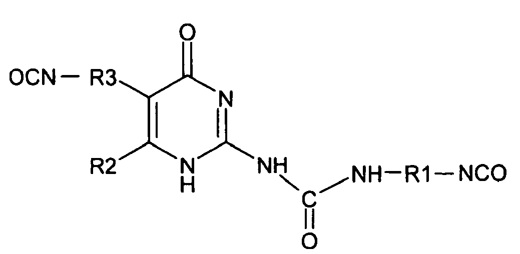 dans laquelle R1, R2 et R3 sont tels que définis dans l'une des revendications précédentes.

12. Composition selon l'une des revendications précédentes, dans laquelle le groupe de jonction est choisi parmi les groupes suivants :

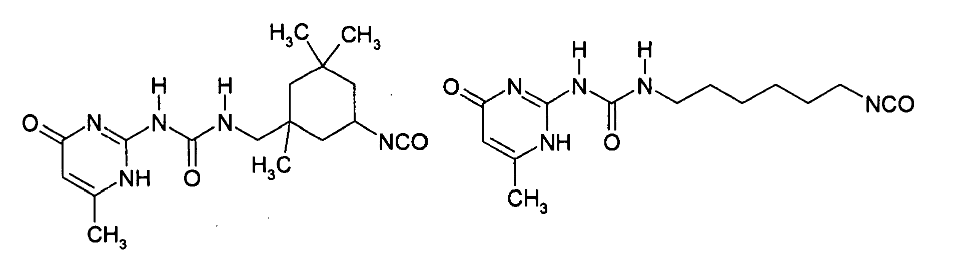

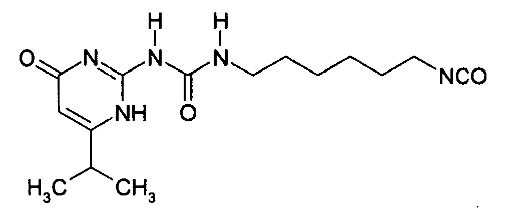

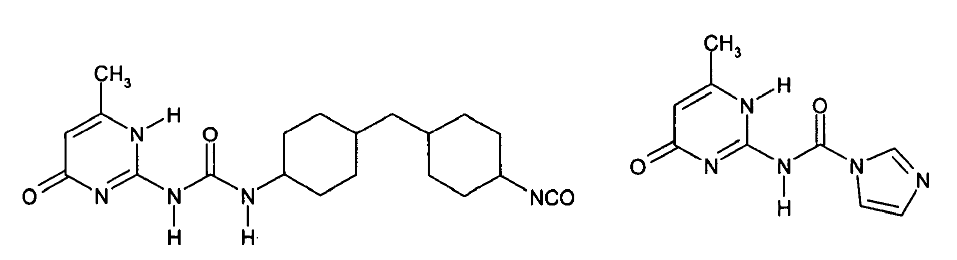

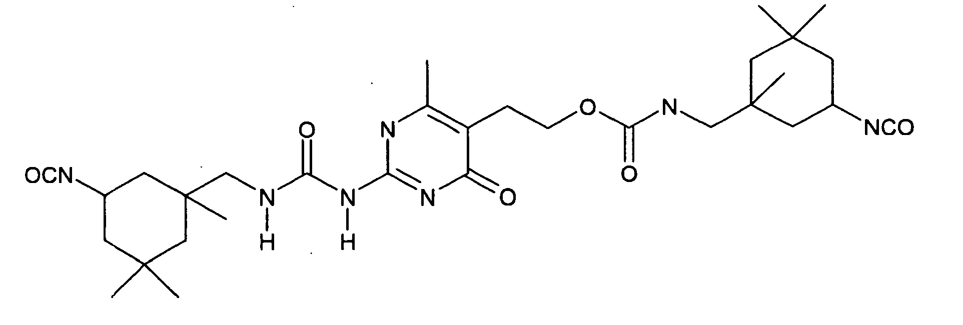

13. Composition selon l'une des revendications précédentes, dans laquelle les composés sont choisis parmi ceux répondant aux structures suivantes :

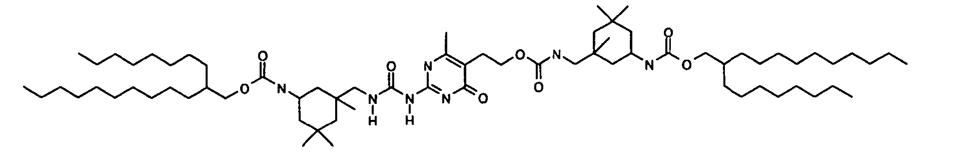

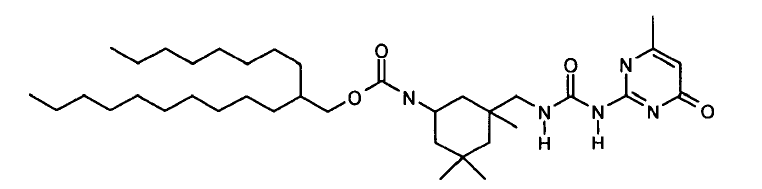

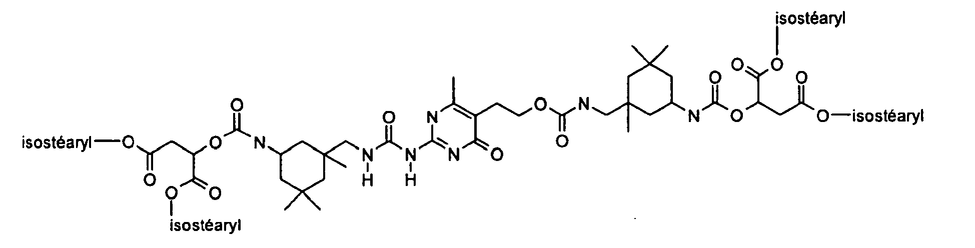

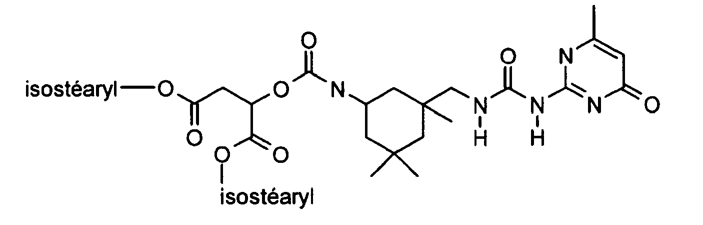

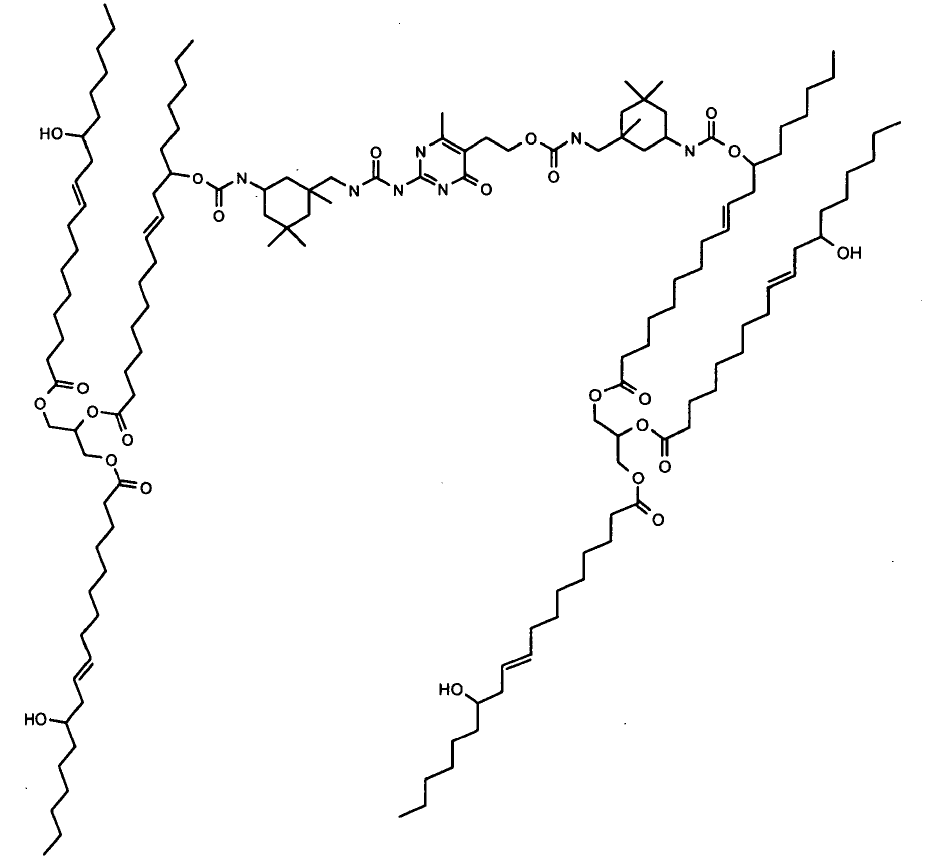

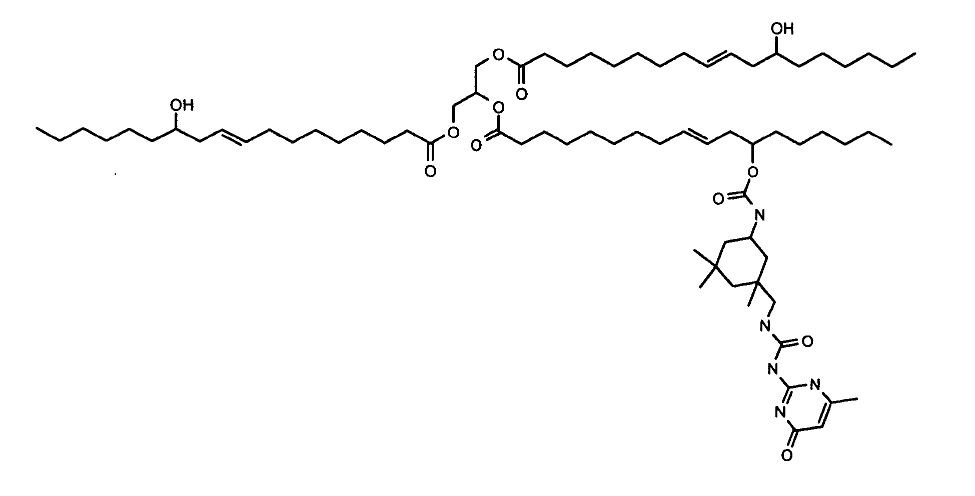

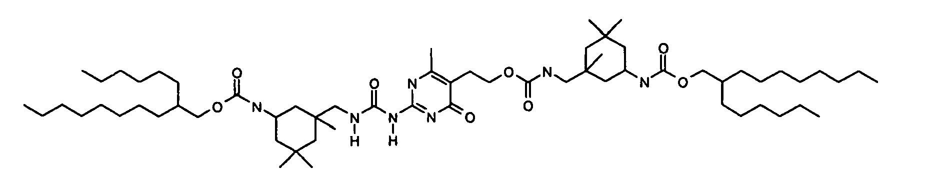

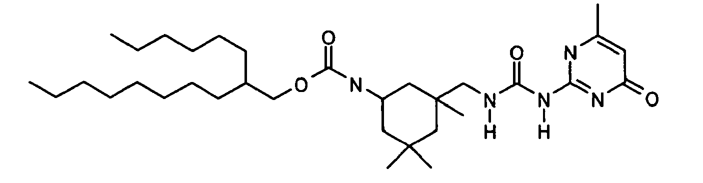

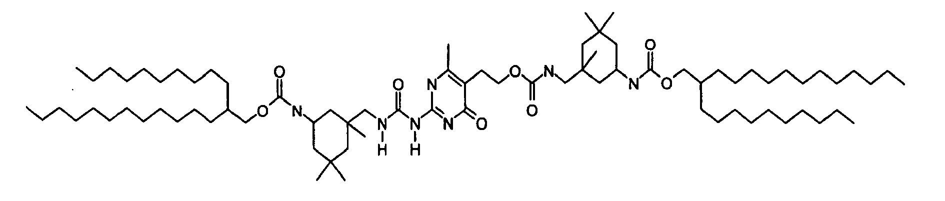

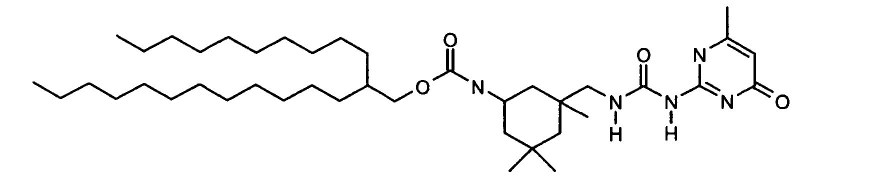

14. Composition selon l'une des revendications précédentes, dans laquelle la masse moléculaire moyenne en nombre (Mn) du composé est comprise entre 180 à 8000.

15. Composition selon l'une des revendications précédentes, dans laquelle la quantité de composé présent dans la composition est comprise entre 5 et 80% en poids, par rapport au poids de la composition cosmétique.

16. Composition selon l'une des revendications précédentes, se présentant sous la forme d'une composition de soin et/ou de maquillage de la peau du corps ou du visage, des lèvres, des cils, des sourcils, ou des ongles; d'un produit solaire ou autobronzant.

17. Procédé de traitement cosmétique des matières kératiniques, notamment de la peau du corps ou du visage, des lèvres, des ongles, et/ou des cils, comprenant l'application sur lesdites matières d'une composition cosmétique telle que définie selon l'une quelconque des revendications 1 à 16.

## Claims

1. Cosmetic composition comprising, in a cosmetically acceptable medium, a compound that may be obtained by reaction between:
- an oil bearing at least one nucleophilic and/or electrophilic reactive function, and
- a junction group capable of establishing hydrogen bonds with one or more partner junction groups, each junction group pairing involving at least 3 hydrogen bonds, the said junction group bearing at least one reactive function capable of reacting with the reactive function borne by the oil, the said junction group also comprising at least one unit of formula (I) or (II): 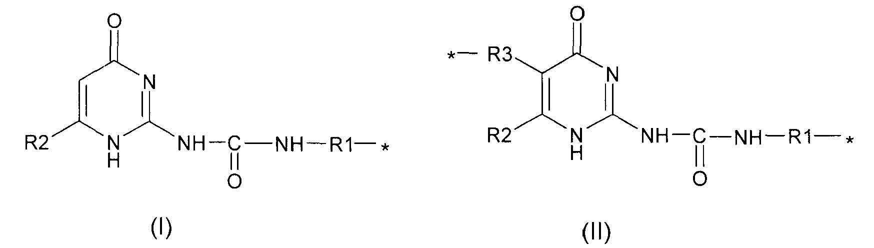 in which:
- R1 and R3, which may be identical or different, represent a divalent carbon-based radical chosen from (i) a linear or branched C₁-C₃₂ alkyl group, (ii) a C₄-C₁₆ cycloalkyl group and (iii) a C₄-C₁₆ aryl group; optionally comprising 1 to 8 heteroatoms chosen from O, N, S, F, Si and P; and/or optionally substituted with an ester or amide function or with a C₁-C₁₂ alkyl radical; or a mixture of these groups;
- R2 represents a hydrogen atom or a linear, branched or cyclic, saturated or unsaturated, optionally aromatic, C1-C32 carbon-based radical, which may comprise one or more heteroatoms chosen from O, N, S, F, Si and P.

2. Composition according to Claim 1, in which the oil bearing the reactive function is chosen, alone or as a mixture, from:
(i) saturated or unsaturated, linear, branched or cyclic fatty alcohols containing 6 to 50 carbon atoms, comprising one or more OH; optionally comprising one or more NH₂ ;
(ii) esters and ethers bearing at least one free OH;
(iii) hydroxylated natural oils, modified natural oils and plant oils.

3. Composition according to either of the preceding claims, in which the oil bearing the reactive function is chosen, alone or as a mixture, from:
- saturated or unsaturated, linear or branched C6-C50 monoalcohols;
- saturated or unsaturated, linear or branched C6-C50 diols;
- saturated or unsaturated, linear or branched C6-C50 triols;
- pentaerythritol partial esters;
- dipentaerythritol diesters, triesters, tetraesters or pentaesters;
- trimethylolpropane monoesters and diesters;
- bis(trimethylolpropane) monoesters, diesters and triesters;
- partial monoesters or polyesters of glycerol or of polyglycerols;
- propylene glycol monoesters;
- diol dimer monoesters;
- glycerol ethers;
- esters between a hydroxylated monocarboxylic, dicarboxylic or tricarboxylic acid and monoalcohols;
- citric acid esters, and especially C4-40 alkyl citrates, such as triisostearyl citrate, triisocetyl citrate and triisoarachidyl citrate;
- triglyceryl esters bearing one or more OHs;
- hydrogenated or non-hydrogenated castor oil, and derivatives thereof;
- modified epoxidized oils, the modification consisting in opening the epoxy function to obtain a diol; hydroxylated soybean oils (directly hydroxylated or epoxidized beforehand).

4. Composition according to one of the preceding claims, in which the oil is a glossy oil, i.e. an oil with a refractive index of greater than or equal to 1.46 at 25°C.

5. Composition according to one of the preceding claims, in which the oil has a molar mass (Mw) of between 150 and 6000.

6. Composition according to one of the preceding claims, in which the oil is chosen from 2-octyldodecanol, diisostearyl malate, 2-butyloctanol, 2-hexyldecanol, 2-decyltetradecanol; hydrogenated or non-hydrogenated castor oil, and also derivatives thereof; hydroxylated modified soybean oil; polyglyceryl-3 diisostearate, phytanetriol, oleyl alcohol, octyl hydroxystearate, polyglyceryl-2 isostearate, polyglyceryl-2 diisostearate, and mixtures thereof.

7. Composition according to one of the preceding claims, in which, in the junction group, the radical R1 represents:
- a linear or branched, divalent C2-C12 alkylene group, especially a 1,2-ethylene, 1,6-hexylene, 1,4-butylene, 1,6-(2,4,4-trimethylhexylene), 1,4-(4-methylpentylene), 1,5-(5-methylhexylene), 1,6-(6-methylheptylene), 1,5-(2,2,5-trimethylhexylene) or 1,7-(3,7-dimethyloctylene) group;
- a divalent C4-C12 cycloalkylene or arylene group, chosen especially from the following radicals: -isophorone-, tolylene, 2-methyl-1,3-phenylene, 4-methyl-1,3-phenylene; 4,4'-methylenebiscyclohexylene; 4,4-bisphenylenemethylene; or of structure:

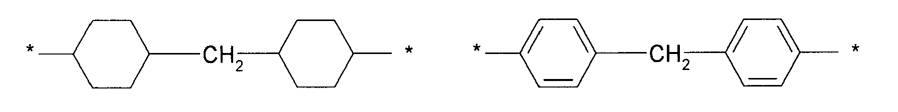

8. Composition according to one of the preceding claims, in which, in the junction group, the radical R2 represents H or:
- a C₁-C₃₂ alkyl group;
- a C₄-C₁₂ cycloalkyl group;
- a C₄-C₁₂ aryl group;
- a (C₄-C₁₂) aryl (C₁-C₁₈) alkyl group;
- a C₁-C₄ alkoxy group;
- an aryl(C₁-C₄)alkoxy group;
- a C₄-C₁₂ heterocycle;
or a combination of these radicals, which may be optionally substituted with an amino, ester and/or hydroxyl function.

9. Composition according to one of the preceding claims, in which, in the junction group, the radical R3 represents a divalent radical -R'3-O-C(O)-NH-R'4- in which R'3 and R'4, which may be identical or different, represent a divalent carbon-based radical chosen from a linear or branched C₁-C₃₂ alkyl group, a C₄-C₁₆ cycloalkyl group and a C₄-C₁₆ aryl group; or a mixture thereof.

10. Composition according to one of the preceding claims, in which, in the junction group,
(a) in formula (I), the following apply:
- R₁ = -isophorone- and R2 = methyl,
- R₁ = - (CH₂)₆- and R2 = methyl,
- R₁ = - (CH₂)₆ - and R2 = isopropyl, or
- R₁ = 4,4'-methylenebiscyclohexylene and R2 = methyl,
or
(b) in formula (II), R1 represents the -isophorone- radical, R2 = methyl and R3 = -(CH₂)₂OCO-NH-isophorone-.

11. Composition according to one of the preceding claims, in which the junction group has the formula: 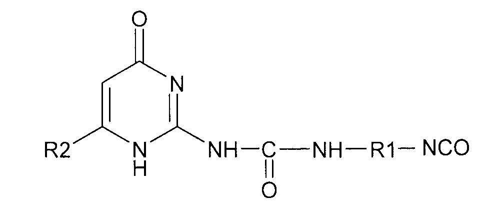 or the formula: 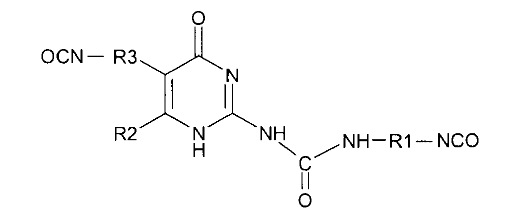 in which R1, R2 and R3 are as defined in one of the preceding claims.

12. Composition according to one of the preceding claims, in which the junction group is chosen from the following groups:

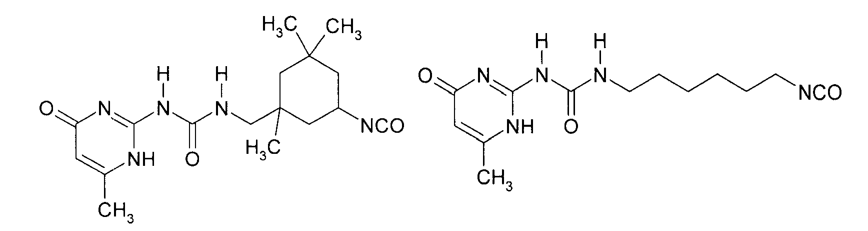

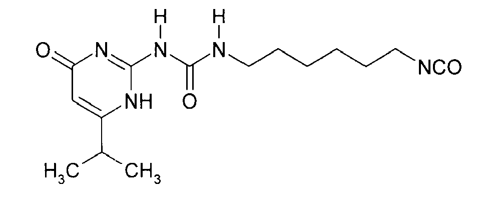

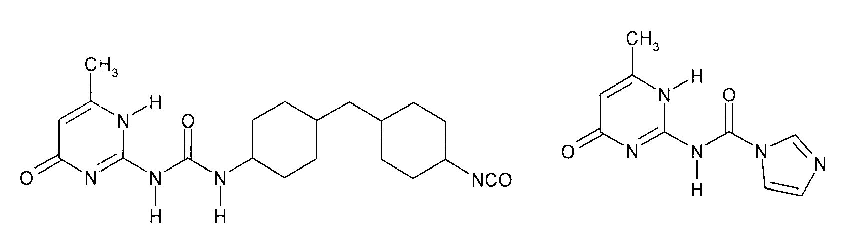

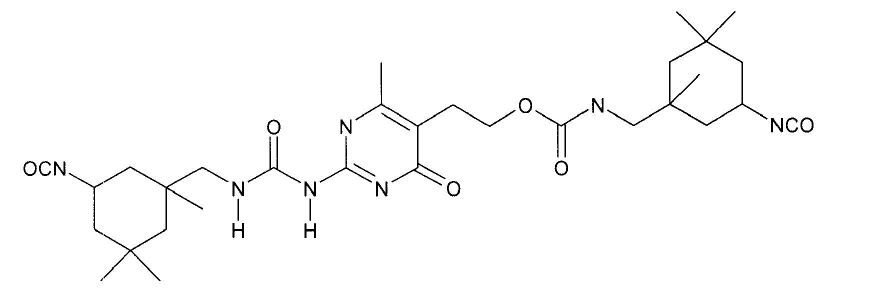

13. Composition according to one of the preceding claims, in which the compounds are chosen from those corresponding to the following structures:

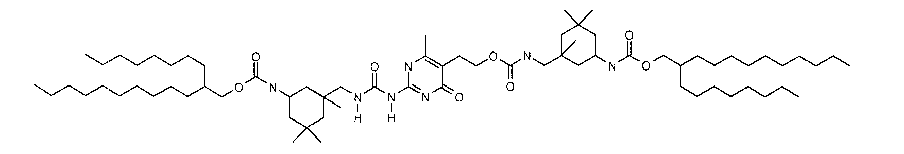

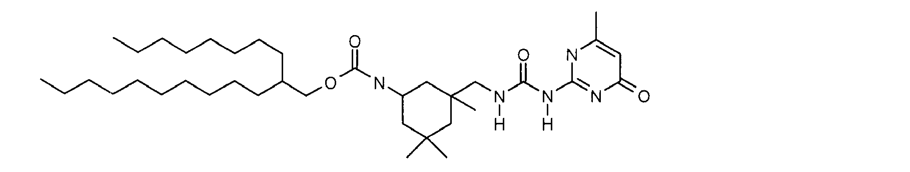

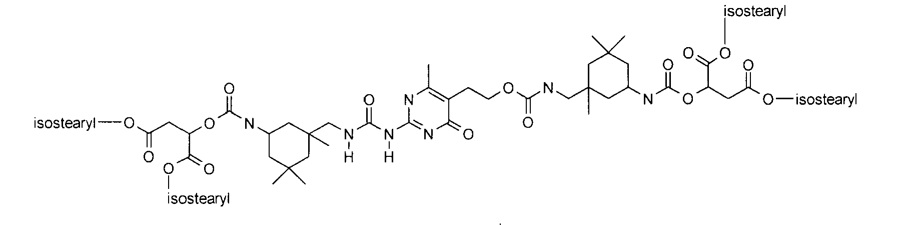

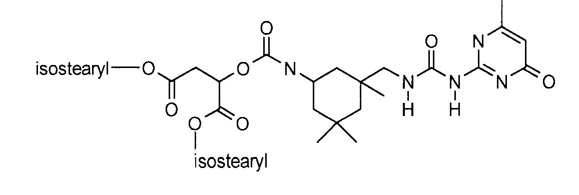

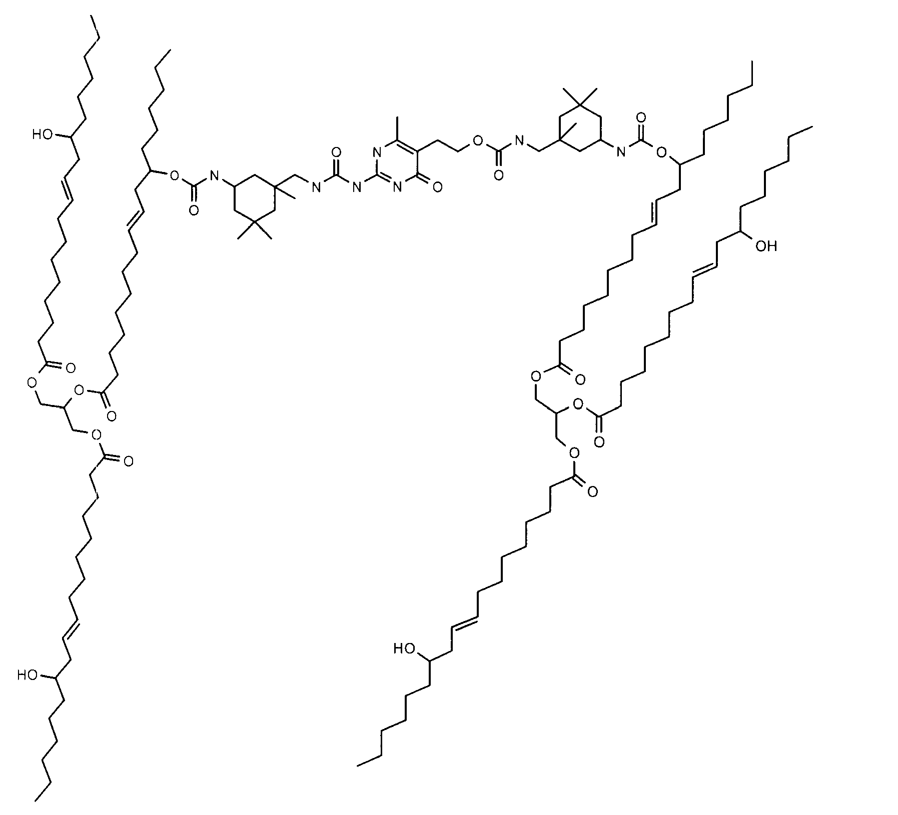

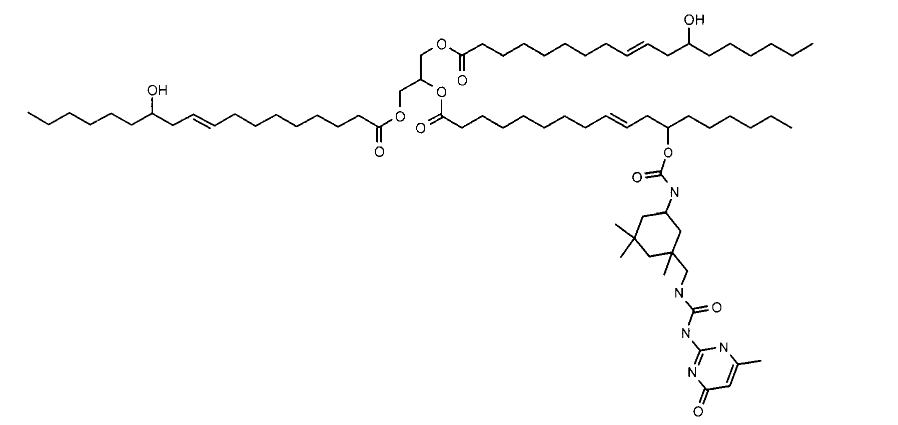

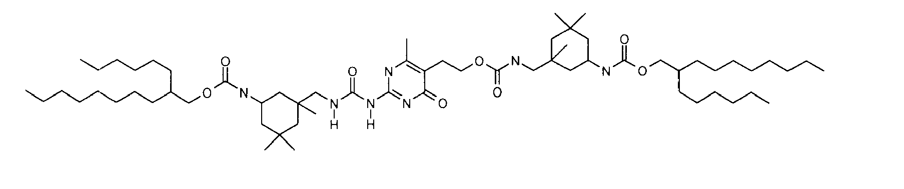

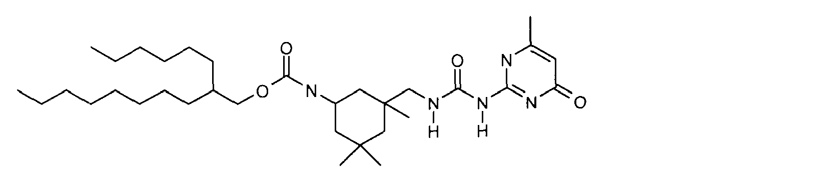

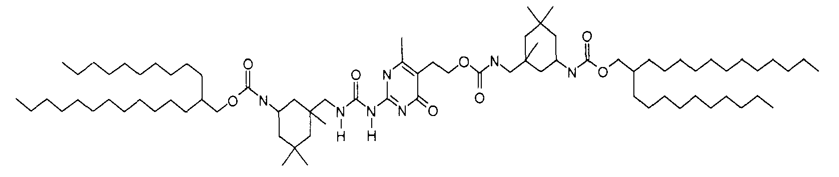

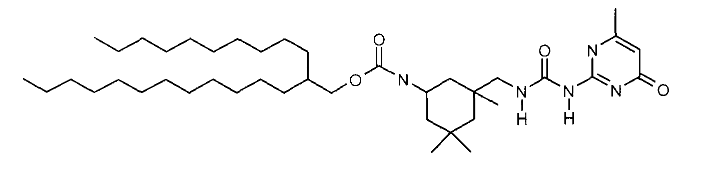

14. Composition according to one of the preceding claims, in which the number-average molecular mass (Mn) of the compound is between 180 and 8000.

15. Composition according to one of the preceding claims, in which the amount of compound present in the composition is between 5% and 80% by weight relative to the weight of the cosmetic composition.

16. Composition according to one of the preceding claims, which is in the form of a care and/or makeup composition for bodily or facial skin, the lips, the eyelashes, the eyebrows or the nails; an antisun or self-tanning product.

17. Cosmetic process for treating keratin materials, especially bodily or facial skin, the lips, the nails and/or the eyelashes, comprising the application to the said materials of a cosmetic composition as defined according to any one of Claims 1 to 16.

## Patentansprüche

1. Kosmetische Zusammensetzung, die in einem kosmetisch unbedenklichen Medium eine Verbindung umfaßt, die durch Reaktion von:
- einem Öl mit mindestens einer nucleophilen und/oder elektrophilen reaktiven Funktion und
- einer Verbindungsgruppe, die zur Ausbildung von Wasserstoffbrückenbindungen mit einer oder mehreren Partner-Verbindungsgruppen befähigt ist, wobei an jeder Paarung einer Verbindungsgruppe mindestens 3 Wasserstoffbrückenbindungen beteiligt sind, wobei die Verbindungsgruppe mindestens eine reaktive Funktion trägt, die mit der reaktiven Funktion des Öls reagieren kann, wobei die Verbindungsgruppe außerdem mindestens eine Einheit der Formel (I) oder (II): 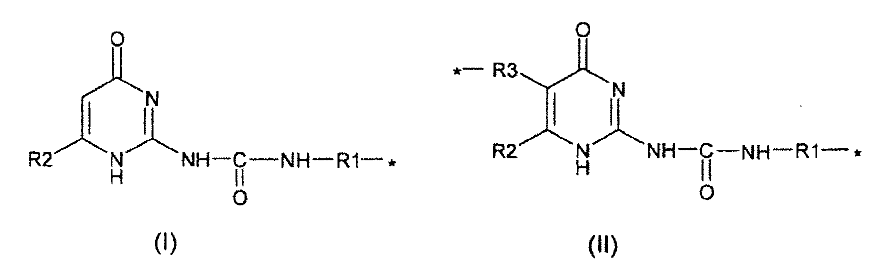 umfaßt, worin:
- R1 und R3 gleich oder verschieden sind und für einen zweiwertigen Kohlenstoffrest, der unter (i) einer linearen oder verzweigten C₁-C₃₂-Alkylgruppe, (ii) einer C₄-C₁₆-Cycloalkylgruppe und (iii) einer C₄-C₁₆-Arylgruppe, die gegebenenfalls 1 bis 8 unter O, N, S, F, Si und P ausgewählte Heteroatome umfaßt und/oder gegebenenfalls durch eine Ester- oder Amidfunktion oder einen C₁-C₁₂-Alkylrest substituiert ist, ausgewählt ist, oder eine Mischung dieser Gruppen stehen;
- R2 für ein Wasserstoffatom oder einen linearen, verzweigten oder cyclischen, gesättigten oder ungesättigten, gegebenenfalls aromatischen C₁-C₃₂-Kohlenstoffrest, der ein oder mehrere unter O, N, S, F, Si und P ausgewählte Heteroatome umfassen kann, steht;
erhältlich ist.

2. Zusammensetzung nach Anspruch 1, wobei das Öl mit der reaktiven Funktion, allein oder als Mischung, unter:
(i) linearen, verzweigten oder cyclischen, gesättigten oder ungesättigten Fettalkoholen mit 6 bis 50 Kohlenstoffatomen und 1 oder mehreren OH und gegebenenfalls einem oder mehreren NH₂;
(ii) Estern und Ethern mit mindestens einer freien OH-Gruppe ;
(iii) hydroxylgruppenhaltigen natürlichen Ölen, modifizierten natürlichen Ölen und Pflanzenölen ausgewählt ist.

3. Zusammensetzung nach einem der vorhergehenden Ansprüche, wobei das Öl mit der reaktiven Funktion, allein oder als Mischung, unter:
- linearen oder verzweigten, gesättigten oder ungesättigten C₆-C₅₀-Monoalkoholen;
- linearen oder verzweigten, gesättigten oder ungesättigten C₆-C₅₀-Diolen;
- linearen oder verzweigten, gesättigten oder ungesättigten C₆-C₅₀-Triolen;
- Pentaerythritpartialestern;
- Dipentaerythritdiestern, -triestern, -tetraestern und -pentaestern;
- Trimethylolpropanmonoestern und -diestern;
- Di(trimethylolpropan)monoestern, -diestern und
- triestern;
- Mono- oder Polypartialestern von Glycerin oder Polyglycerinen;
- Propylenglykolmonoestern;
- Dioldimermonoestern;
- Glycerinethern;
- Estern von hydroxylgruppenhaltigen Mono-, Di- oder Tricarbonsäuren und Monoalkoholen;
- Triglycerylestern mit einem oder mehreren OH;
- gegebenenfalls hydriertem Ricinusöl sowie Derivaten davon;
- modifizierten epoxidierten Ölen, wobei die Modifizierung in der Öffnung der Epoxidfunktion zu einem Diol besteht; hydroxylierten Sojaölen (direkthydroxyliert oder vorepoxidiert) ausgewählt ist.

4. Zusammensetzung nach einem der vorhergehenden Ansprüche, wobei es sich bei dem Öl um ein Glanzöl, d.h. ein Öl mit einem Brechungsindex größer gleich 1,46 bei 25°C, handelt.

5. Zusammensetzung nach einem der vorhergehenden Ansprüche, wobei das Öl eine Molmasse (Mw) zwischen 150 und 6000 g/mol aufweist.

6. Zusammensetzung nach einem der vorhergehenden Ansprüche, wobei das Öl unter 2-Octyldodecanol, Diisostearylmalat, 2-Butyloctanol, 2-Hexyldecanol, 2-Decyltetradecanol; gegebenenfalls hydriertem Ricinusöl sowie Derivaten davon; hydroxyliertem modifiziertem Sojaöl; Polyglyceryl-3-diisostearat, Phytantriol, Oleylalkohol, Octylhydroxystearat, Polyglyceryl-2-isostearat, Polyglyceryl-2-diisostearat und Mischungen davon ausgewählt ist.

7. Zusammensetzung nach einem der vorhergehenden Ansprüche, wobei der Rest R1 in der Verbindungsgruppe für:
- eine lineare oder verzweigte zweiwertige C₂-C₁₂-Alkylengruppe, insbesondere eine 1,2-Ethylen-, 1,6-Hexylen-, 1,4-Butylen-, 1,6-(2,4,4-Trimethylhexylen)-, 1,4-(4-Methylpentylen)-, 1,5-(5-Methylhexylen)-, 1,6-(6-Methylheptylen)-, 1,5-(2,2,5-Trimethylhexylen)- oder 1,7-(3,7-Dimethyl-octylen) gruppe;
- eine zweiwertige C₄-C₁₂-Cycloalkylen- oder -Arylengruppe, die insbesondere unter den folgenden Resten ausgewählt ist: -Isophoron-, Toluylen, 2-Methyl-1,3-phenylen, 4-Methyl-1,3-phenylen, 4,4'-Methylenbiscyclohexylen, 4,4-Bisphenylenmethylen oder der Struktur: 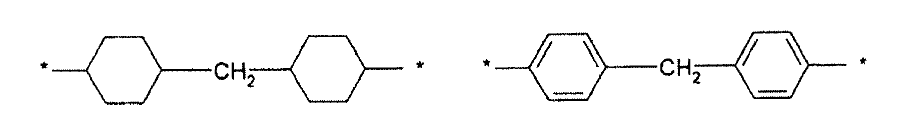 steht.

8. Zusammensetzung nach einem der vorhergehenden Ansprüche, wobei der Rest R2 in der Verbindungsgruppe für H oder:
- eine C₁-C₃₂-Alkylgruppe;
- eine C₄-C₁₂-Cycloalkylgruppe;
- eine C₄-C₁₂-Arylgruppe;
- eine C₄-C₁₂-Aryl-C₁-C₁₈-alkylgruppe;
- eine C₁-C₄-Alkoxygruppe;
- eine Aryl-C₁-C₄-alkoxygruppe;
- einen C₄-C₁₂-Heterocyclus
oder eine Kombination dieser Reste, die gegebenenfalls durch eine Amino-, Ester- und/oder Hydroxyfunktion substituiert sein können, steht.

9. Zusammensetzung nach einem der vorhergehenden Ansprüche, wobei der Rest R3 in der Verbindungsgruppe für einen zweiwertigen Rest -R'3-O-C(O)-NH-R'4- steht, worin R'3 und R'4 gleich oder verschieden sind und für einen zweiwertigen Kohlenstoffrest, der unter einer linearen oder verzweigten C₁-C₃₂-Alkylgruppe, einer C₄-C₁₆-Cycloalkylgruppe und einer C₄-C₁₆-Arylgruppe augewählt ist, oder eine Mischung davon stehen.

10. Zusammensetzung nach einem der vorhergehenden Ansprüche, wobei in der Verbindungsgruppe
(a) in Formel (I) folgendes gilt:
- R₁ = -Isophoron- und R2 = Methyl,
- R₁ = - (CH₂)₆ - und R2 = Methyl,
- R₁ = - (CH₂)₆- und R2 = Isopropyl oder
- R₁ = 4,4'-Methylenbiscyclohexylen und R2 = Methyl,
oder
(b) in Formel (II) R1 für den -Isophoron-Rest steht, R2 = Methyl und R3 = -(CH₂)₂OCO-NH-Isophoron-.

11. Zusammensetzung nach einem der vorhergehenden Ansprüche, wobei die Verbindungsgruppe die Formel: 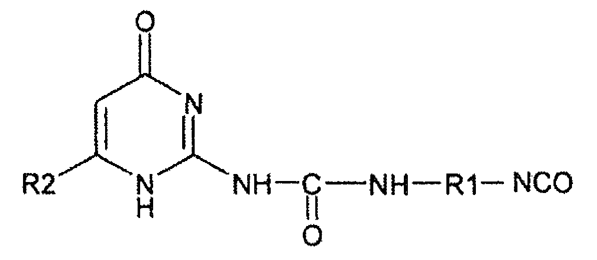 oder die Formel: 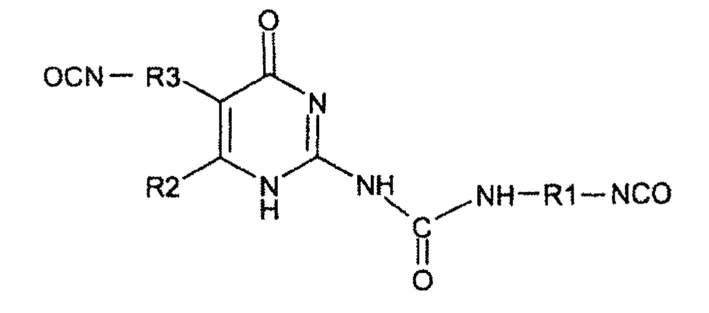 worin R1, R2 und R3 die in einem der vorhergehenden Ansprüche angegebene Bedeutung besitzen, aufweist.

12. Zusammensetzung nach einem der vorhergehenden Ansprüche, wobei die Verbindungsgruppe unter den folgenden Gruppen ausgewählt ist:

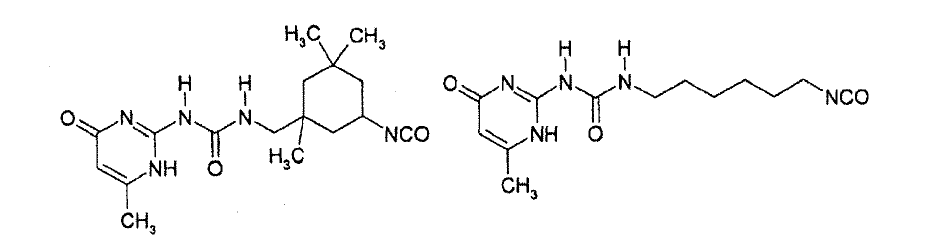

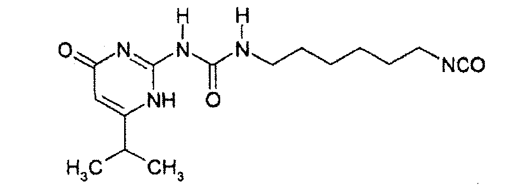

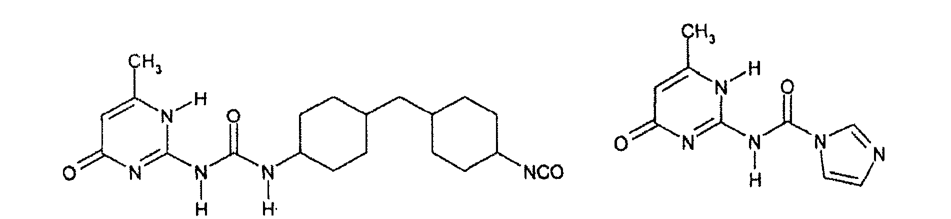

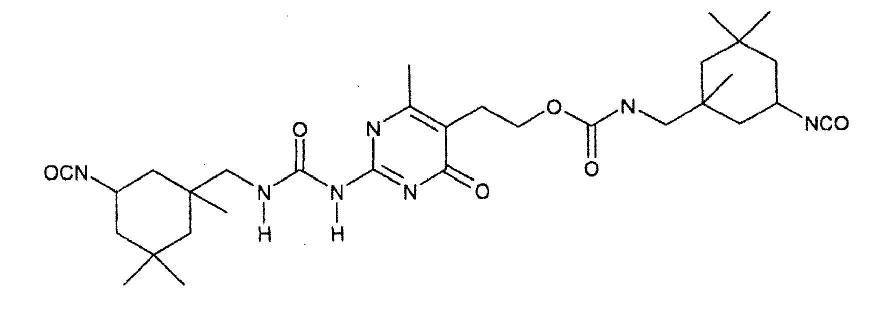

13. Zusammensetzung nach einem der vorhergehenden Ansprüche, wobei die Verbindungen unter denjenigen ausgewählt sind, die den folgenden Strukturen entsprechen:

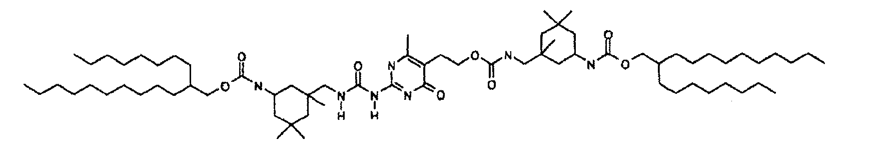

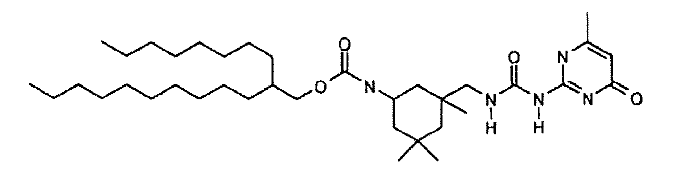

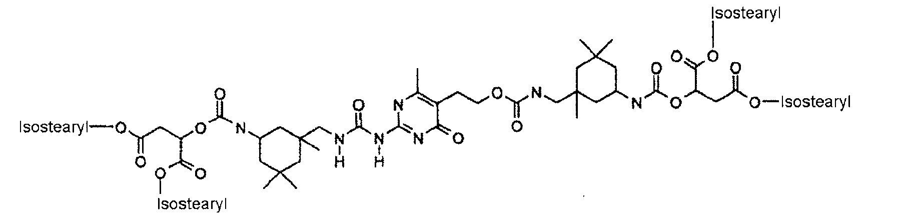

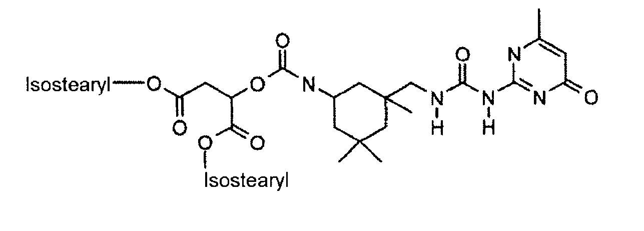

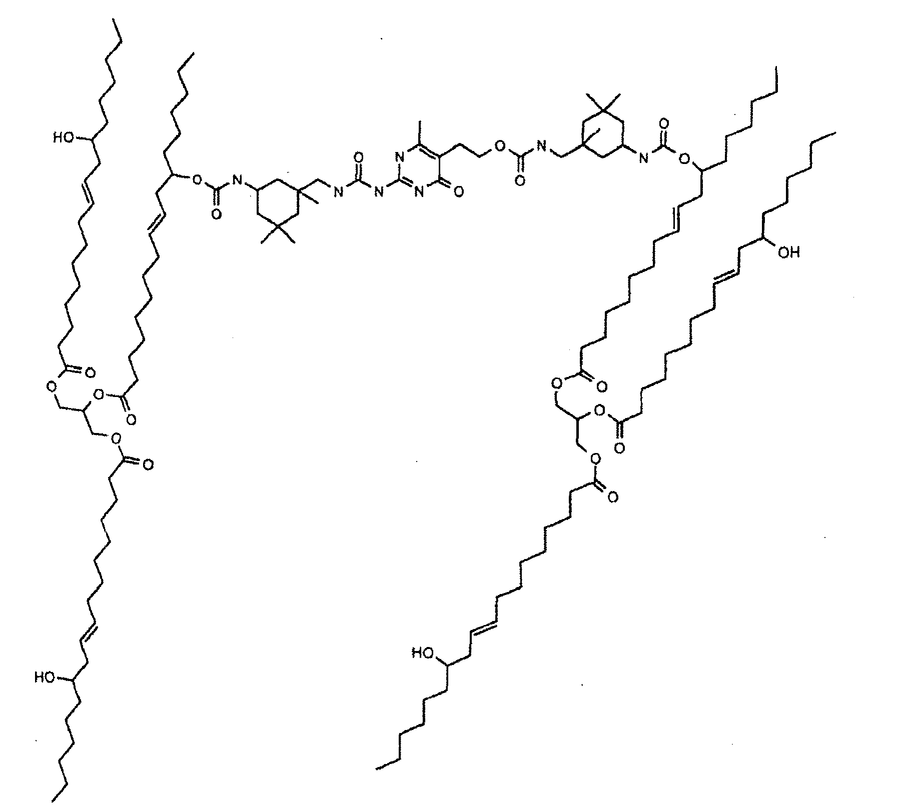

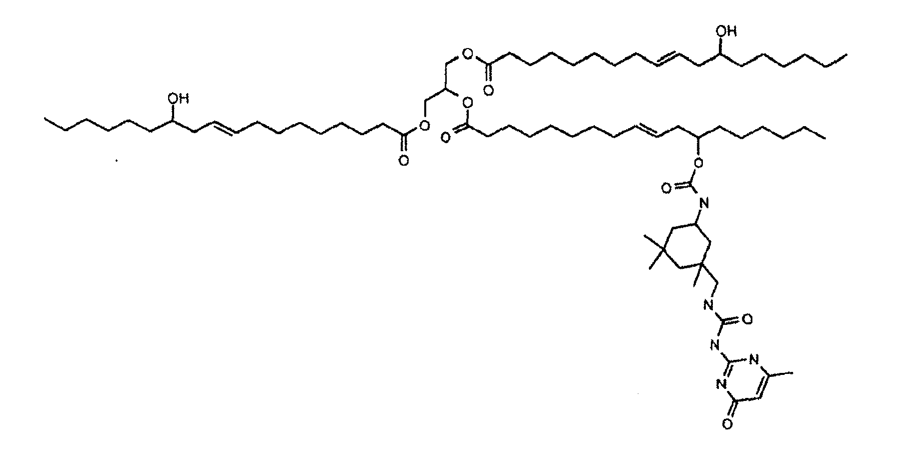

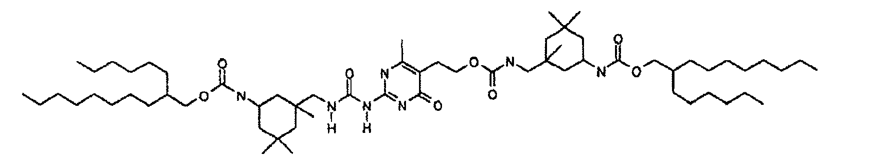

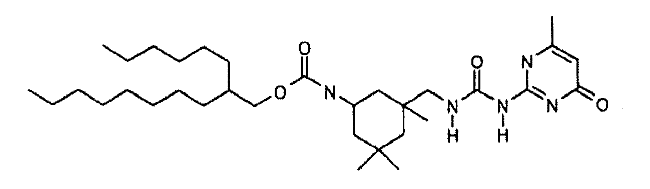

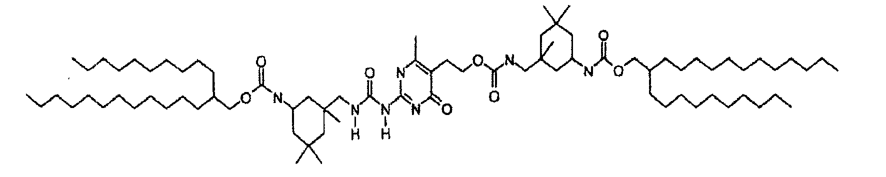

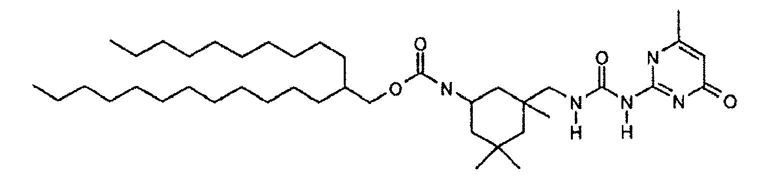

14. Zusammensetzung nach einem der vorhergehenden Ansprüche, wobei die zahlenmittlere Molmasse (Mn) der Verbindung zwischen 180 und 8000 liegt.

15. Zusammensetzung nach einem der vorhergehenden Ansprüche, wobei die in der Zusammensetzung vorliegende Verbindungsmenge zwischen 5 und 80 Gew.-%, bezogen auf das Gewicht der kosmetischen Zusammensetzung, liegt.

16. Zusammensetzung nach einem der vorhergehenden Ansprüche, die in Form einer Pflege- und/oder Makeup-Zusammensetzung für die Körper- oder Gesichtshaut, die Lippen, der Wimpern, der Augenbrauen oder der Nägel, eines Sonnenschutzprodukts oder eines Selbstbräunungsprodukts vorliegt.

17. Verfahren zur kosmetischen Behandlung von Keratinmaterialien, inbesondere der Körper- oder Gesichtshaut, der Lippen, der Nägel und/oder der Wimpern, bei dem man auf die Materialien eine kosmetische Zusammensetzung gemäß einem der Ansprüche 1 bis 16 aufbringt.
